(19) Europäisches Patentamt / European Patent Office / Office européen des brevets

(11) EP 1 734 919 B1

(12) **FASCICULE DE BREVET EUROPEEN**

(45) Date de publication et mention de la délivrance du brevet:
**10.09.2008 Bulletin 2008/37**

(21) Numéro de dépôt: **05739594.9**

(22) Date de dépôt: **18.03.2005**

(51) Int Cl.:
***A61K 8/25*** (2006.01) ***A61K 8/31*** (2006.01)
***A61K 8/91*** (2006.01) ***A61K 8/58*** (2006.01)
***A61Q 19/00*** (2006.01) ***A61Q 19/08*** (2006.01)

(86) Numéro de dépôt international:
**PCT/FR2005/050174**

(87) Numéro de publication internationale:
**WO 2005/092284 (06.10.2005 Gazette 2005/40)**

# (54) PROCEDE DE TRAITEMENT COSMETIQUE D' UNE PEAU RIDEE METTANT EN OEUVRE UNE COMPOSITION COSMETIQUE COMPRENANT UN AGENT TENSEUR ET UNE DISPERSION DE PARTICULES SOLIDES D' UN POLYMERE ACRYLIQUE GREFFE

VERFAHREN FÜR DIE KOSMETISCHE BEHANDLUNG VON FALTIGER HAUT MIT EINER KOSMETISCHEN ZUSAMMENSETZUNG AUS EINEM STRAFFENDEN MITTEL UND EINER DISPERSION AUS FESTEN TEILCHEN EINES GEPFROPFTEN ACRYLPOLYMERS

METHOD FOR THE COSMETIC TREATMENT OF WRINKLED SKIN USING A COSMETIC COMPOSITION CONTAINING A TIGHTENING AGENT AND A DISPERSION OF SOLID PARTICLES OF A GRAFTED ACRYLIC POLYMER

(84) Etats contractants désignés:
**AT BE BG CH CY CZ DE DK EE ES FI FR GB GR HU IE IS IT LI LT LU MC NL PL PT RO SE SI SK TR**

(30) Priorité: **19.03.2004 FR 0450556**

(43) Date de publication de la demande:
**27.12.2006 Bulletin 2006/52**

(73) Titulaire: **L'ORÉAL**
**75008 Paris (FR)**

(72) Inventeurs:
- **CASSIN, Guillaume**
  **F-91140 Villebon Sur Yvette (FR)**
- **VICIC, Marco**
  **F-94360 Bry Sur Marne (FR)**

(74) Mandataire: **Poulin, Gérard**
**BREVALEX**
**3, rue du Docteur Lancereaux**
**75008 Paris (FR)**

(56) Documents cités:
**EP-A- 0 749 747** **EP-A- 0 963 751**
**EP-A- 1 038 519** **EP-A- 1 428 844**
**WO-A-97/35541** **WO-A-20/04055077**
**US-A- 5 219 560** **US-A- 5 262 087**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 734 919 B1

## Description

### DOMAINE TECHNIQUE

La présente invention a trait à un procédé cosmétique pour atténuer les rides d'une peau ridée mettant en oeuvre une composition cosmétique, notamment une composition antirides, comprenant, dans un milieu physiologiquement acceptable adapté à une application topique sur la peau du visage, de 0,1% à 20% en poids d'un agent tenseur, par rapport au poids total de la composition et une dispersion d'un polymère éthylénique particulier apte à engendrer une rémanence de l'effet tenseur engendré par l'agent tenseur susmentionné.

La présente invention a trait à l'utilisation d'une dispersion d'un polymère éthylénique particulier pour améliorer la rémanence de l'effet tenseur procuré par un agent tenseur.

La présente invention a également trait à l'utilisation d'une dispersion d'un polymère éthylénique particulier dans une composition comprenant en tant qu'agent tenseur une dispersion colloïdale de particules inorganiques pour prévenir le blanchiment de la peau.

Le domaine général de l'invention est donc celui du vieillissement de la peau.

Au cours du vieillissement de la peau, il apparaît différents signes se traduisant notamment par une modification de la structure et des fonctions cutanées. L'un de ces principaux signes est l'apparition de ridules et de rides profondes dont l'importance et le nombre croissent avec l'âge. Le microrelief de la peau devient moins régulier et présente un caractère anisotrope.

### ETAT DE LA TECHNIQUE ANTERIEURE

Il est courant de traiter ces signes de vieillissement par des compositions cosmétiques contenant des actifs susceptibles de lutter contre le vieillissement, tels que des $\alpha$-hydroxyacides, des $\beta$-hydroxyacides et des rétinoïdes. Ces actifs agissent notamment sur les rides en éliminant les cellules mortes de la peau et en accélérant le processus de renouvellement cellulaire. Cependant, ces actifs présentent l'inconvénient de n'être efficaces pour le traitement des rides qu'après un certain temps d'application, à savoir un temps pouvant aller de quelques jours à plusieurs semaines.

Or, les besoins actuels tendent de plus en plus vers l'obtention de compositions permettant d'obtenir un effet immédiat, conduisant rapidement à un lissage des rides et/ou ridules et à la disparition, même temporaire, des marques de fatigue. De telles compositions sont des compositions comprenant des agents tenseurs. On précise que, par « agent tenseur » on entend des composés susceptibles d'avoir un effet tenseur, c'est-à-dire pouvant tendre la peau et y faire diminuer voire disparaître de façon immédiate les rides et les ridules.

Ces agents tenseurs peuvent être notamment des polymères d'origine naturelle ou synthétique en dispersion aqueuse, capables de former un film provoquant la rétraction du *stratum corneum,* c'est-à-dire la couche cornée superficielle de l'épiderme. L'utilisation cosmétique ou dermatologique de tels systèmes polymériques pour atténuer les effets du vieillissement de la peau est décrite dans la demande de brevet FR-A-2,758,083 [1] ou dans la demande EP-A-1038519. Ce dernier décrit un polymère siliconé greffé.

L'utilisation de ces systèmes polymériques tenseurs engendre cependant parfois une impression d'inconfort chez certains utilisateurs, notamment ceux présentant une peau fragile. En outre, l'effet tenseur qu'ils procurent ne dure pas très longtemps, dans la mesure où le film formé sur la peau a tendance à se fissurer sous l'effet des mimiques. Ces agents tenseurs forment en effet sur la peau un film relativement rigide et peu déformable.

La demanderesse a constaté de façon surprenante que l'utilisation d'une dispersion d'un polymère éthylénique particulier en association avec un agent tenseur dans une composition cosmétique permettait d'obtenir, après application topique sur une peau ridée, des films présentant un effet tenseur durable, lesdits films étant du point de vue mécanique plus déformables.

En outre, il est apparu à la Demanderesse que cette dispersion de polymères éthyléniques particuliers avaient de plus la propriété d'éviter le blanchiment de la peau consécutif à l'application sur celle-ci de compositions comprenant comme agents tenseurs des dispersions colloïdales de particules inorganiques, en particulier de silice.

### EXPOSÉ DE L'INVENTION

Ainsi, l'invention concerne, selon un premier objet, un procédé cosmétique pour atténuer les rides d'une peau ridée comprenant une étape consistant à appliquer sur ladite peau une composition cosmétique, notamment une composition antirides, comprenant dans un milieu physiologiquement acceptable adapté à une application topique sur la peau du visage:

- de 0,1 à 20% en poids d'au moins un agent tenseur, par rapport au poids total de la composition,et
- au moins une dispersion dans une phase grasse liquide de particules solides d'un polymère éthylénique greffé.

L'utilisation d'une dispersion telle que définie précédemment en association avec un agent tenseur permet de conférer à la composition dans laquelle elle est incluse un effet tenseur rémanent, c'est-à-dire un effet tenseur qui présente une certaine durabilité dans le temps, la dispersion assurant un rôle d'agent renforçateur du film tenseur. Des tests visant à mettre en évidence cette propriété de rémanence d'une telle association sont exposés dans la partie expérimentale de cette description. On précise que cette rémanence est justifiée dans le cadre de cette invention par l'amélioration des propriétés mécaniques du film tenseur.

Avant d'entrer plus en détail dans la description, nous proposons les définitions suivantes.

Par agent tenseur, on entend, généralement, selon l'invention, tout agent produisant à une concentration de 7% dans l'eau une rétraction du *stratum corneum* isolé, mesuré avec un extensomètre, de plus de 1%, et de préférence de plus de 1,5% à 30°C sous une humidité relative de 40%.

Le protocole de détermination de rétraction du *stratum corneum* est le suivant :

Le pouvoir tenseur des agents tenseurs décrits dans le présent document a été mesuré avec un extensomètre.

Le principe de la méthode consiste à mesurer la longueur d'une éprouvette de *stratum corneum* isolé à partir de peau humaine provenant d'une opération chirurgicale, avant et après traitement avec les compositions à tester.

Pour ce faire, l'éprouvette est placée entre les deux mâchoires de l'appareil dont l'une est fixe et l'autre mobile, dans une atmosphère à 30°C et à 40% d'humidité relative. On exerce une traction sur l'éprouvette, et on enregistre la courbe de la force (en grammes) en fonction de la longueur (en millimètres), la longueur zéro correspondant au contact entre les deux mors de l'appareil. On trace ensuite la tangente à la courbe dans sa région linéaire. L'intersection de cette tangente avec l'axe des abscisses correspond à la longueur apparente $L_0$ de l'éprouvette à force nulle. On détend ensuite l'éprouvette puis on applique sur le *stratum corneum* 2 mg/cm$^2$ de la composition à tester (solution à 7% de l'agent tenseur considéré). Après 15 minutes de séchage, les étapes ci-dessus sont à nouveau mises en oeuvre pour déterminer la longueur $L_1$ de l'éprouvette après traitement. Le pourcentage de rétraction est défini par : % rétraction = 100 x $(L_1-L_0)/L_0$. Pour caractériser un effet tenseur, ce pourcentage doit être négatif et l'effet tenseur est d'autant plus important que la valeur absolue du pourcentage de rétraction est élevée.

Ledit agent tenseur, dans le cadre de cette invention, peut notamment être choisi parmi :

a) les polymères synthétiques;
b) les polymères d'origine naturelle ;
c) les silicates mixtes ;
d) les microparticules de cire ;
e) les particules colloïdales de charges inorganiques

et les mélanges de ceux-ci.

a) Les polymères synthétiques.

Les polymères synthétiques utilisables en tant qu'agent tenseur peuvent être choisis parmi :

- les polymères et copolymères de polyuréthanne ;
- les polymères et copolymères acryliques ;
- les polymères d'acide isophtalique sulfoné ;
- les polymères siliconés greffés ;
- les polymères hydrosolubles ou hydrodispersibles comprenant des unités hydrosolubles ou hydrodispersibles et des unités à LCST ;
- les polymères éthyléniques séquencés linéaires filmogènes non élastomères et non hydrosolubles présentant un module dynamique de conservation E' à 1Hz et à 22°C supérieur à 200 MPa ;
- une dispersion dans une phase grasse liquide de particules solides d'un polymère éthylénique greffé présentant une température de transition vitreuse supérieure à 40°C ;
- et les mélanges de ceux-ci.

Les copolymères de polyuréthanne, les copolymères acryliques et les autres polymères synthétiques selon l'invention peuvent notamment être choisis parmi les polycondensats, les polymères hybrides et les réseaux de polymères interpénétrés (IPNs).

Par "réseau de polymères interpénétrés" (IPN) au sens de la présente invention, on entend un mélange de deux polymères enchevêtrés, obtenu par polymérisation et/ou réticulation simultanée de deux types de monomères, le mélange obtenu ayant une température de transition vitreuse unique.

Des exemples d'IPNs convenant à une mise en oeuvre dans la présente invention, ainsi que leur procédé de préparation, sont décrits dans les brevets US-6,139,322 [2] et US-6,465,001 [3], par exemple.

De préférence, l'IPN selon l'invention comprend au moins un polymère polyacrylique et, plus préférentiellement, il comprend en outre au moins un polyuréthanne ou un copolymère de fluorure de vinylidène et d'hexafluoropropylène.

Selon une forme d'exécution préférée, l'IPN selon l'invention comprend un polymère polyuréthanne et un polymère polyacrylique. De tels IPNs sont notamment ceux de la série Hybridur qui sont disponibles dans le commerce auprès de la société AIR PRODUCTS.

Un IPN particulièrement préféré se trouve sous la forme d'une dispersion aqueuse de particules ayant une taille moyenne, en poids, comprise entre 90 et 110 nm et une taille moyenne, en nombre, d'environ 80 nm. Cet IPN a de préférence une température de transition vitreuse, Tg, qui va d'environ -60°C à +100°C. Un IPN de ce type est notamment commercialisé par la société AIR PRODUCTS sous la dénomination commerciale Hybridur X-01602. Un autre IPN convenant à une utilisation dans la présente invention est référencé Hybridur X18693-21.

D'autres IPNs convenant à une mise en oeuvre dans la présente invention comprennent les IPNs constitués du mélange d'un polyuréthanne avec un copolymère de fluorure de vinylidène et d'hexafluoropropylène. Ces IPNs peuvent notamment être préparés comme décrit dans le brevet US-5,349,003 [4]. En variante, ils sont disponibles dans le commerce sous forme de dispersion colloïdale dans l'eau, dans un rapport du copolymère fluoré au polymère acrylique comprise entre 70:30 et 75:25, sous les dénominations commerciales KYNAR RC-10,147 et KYNAR RC-10,151 auprès de la société ATOFINA.

Des exemples de polymères siliconés greffés sont indiqués dans la demande EP-1 038 519 [5], qui est incorporée ici par référence.

Un polymère siliconé greffé pouvant être utilisé dans la cadre de cette invention comprend une portion polysiloxane et une portion constituée d'une chaîne organique non siliconée, l'une des deux portions constituant la chaîne principale du polymère et l'autre étant greffée sur ladite chaîne principale.

Dans une première variante où ledit polymère est un polymère à squelette organique non-siliconé greffé par au moins un monomère contenant un polysiloxane, il s'agit de préférence d'un copolymère greffé siliconé comprenant :

a) au moins un monomère (A) lipophile à insaturation éthylénique, polymérisable par voie radicalaire ;
b) au moins un monomère (B) hydrophile polaire à insaturation éthylénique, copolymérisable avec le ou les monomères du type (A) ; tel que (A) + (B) varie de 99,99% à 50% en poids ; et
c) de 0,01 à 50 % en poids d'au moins un macromère polysiloxane (C) de formule (I) suivante :

$$X(Y)_nSi(R)_{3-m}\,Z_m \qquad (I)$$

dans laquelle :

X désigne un groupe à insaturation éthylénique copolymérisable avec les monomères (A) et (B) ;
Y désigne un groupe de liaison divalent ;
R désigne un hydrogène, un groupe hydroxyle, un groupe alkyle ou alkylamino ou alcoxy en $C_1$-$C_6$, ou un groupe aryle en $C_6$-$C_{12}$ ;
Z désigne un motif polysiloxane ayant un poids moléculaire moyen en nombre d'au moins 50;
n est 0 ou 1 et m est un entier allant de 1 à 3 ; les pourcentages étant calculés par rapport au poids total des monomères (A), (B) et (C).

Le monomère (A) peut être choisi dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-méthyl perflurooctane sulfonamido)-éthylacrylate, le 2-(N-butylperflurooctane sulfonamido)-éthylacrylate et leurs mélanges.

Le monomère (B) peut être choisi dans le groupe constitué par l'acide acrylique, le N,N-diméthylacrylamide, le méthacrylate de diméthylaminoéthyle, le méthacrylate de diméthylaminoéthyle quaternisé, la vinylpyrrolidone et leurs mélanges.

Le macromonomère polysiloxane (C) a par exemple pour formule la formule (II) suivante :

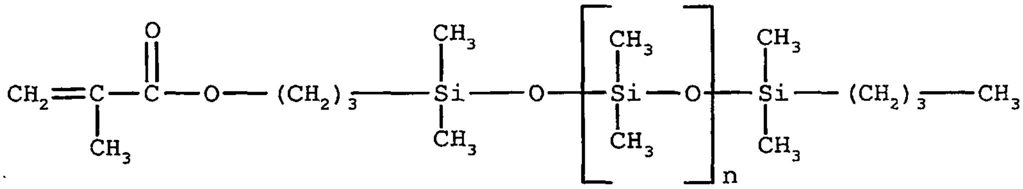

(II)

avec n étant un nombre entier allant de 1 à 700.

Dans une seconde variante préférée, ledit polymère est un polymère à squelette polysiloxanique greffé par au moins un monomère organique non-siliconé.

Ce polymère résulte avantageusement de la copolymérisation radicalaire entre d'une part au moins un monomère organique anionique non-siliconé présentant une insaturation éthylénique (choisi par exemple parmi les acides (méth)acryliques et leurs sels) et/ou un monomère organique hydrophobe non-siliconé présentant une insaturation éthylénique (choisi par exemple parmi les esters d'acide(méth)acrylique d'alcanols) et d'autre part une silicone présentant dans sa chaîne au moins un groupement fonctionnel capable de venir réagir sur lesdites insaturations éthyléniques desdits monomères non-siliconés en formant une liaison covalente.

Dans ce cas, ledit polymère siliconé comporte de préférence dans sa structure le motif de formule (III) suivant :

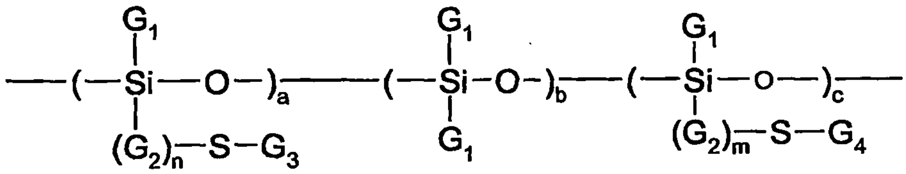

(III)

dans lequel les radicaux $G_1$, identiques ou différents, représentent un hydrogène ou un radical alkyle en $C_1$-$C_{10}$ ou encore un radical phényle ; les radicaux $G_2$, identiques ou différents, représentent un groupe alkylène en $C_1$-$C_{10}$ ; $G_3$ représente un reste polymérique résultant de l'(homo)polymérisation d'au moins un monomère anionique à insaturation éthylénique ; $G_4$ représente un reste polymérique résultant de l'(homo)polymérisation d'au moins un monomère hydrophobe à insaturation éthylénique ; m et n sont, indépendamment l'un de l'autre, égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

Plus préférentiellement encore, ledit motif de formule (III) présente au moins l'une, et de préférence l'ensemble, des caractéristiques suivantes :

- les radicaux $G_1$ désignent un radical alkyle en $C_1$-$C_{10}$ ;
- n est non nul, et les radicaux $G_2$ représentent un radical divalent en $C_1$-$C_3$ ;
- $G_3$ représente un radical polymérique résultant de l'(homo)polymérisation d'au moins un monomère du type acide carboxylique à insaturation éthylénique ;
- $G_4$ représente un radical polymérique résultant de l'(homo)polymérisation d'au moins un monomère du type (méth) acrylate d' alkyle en $C_1$-$C_{10}$.

Il peut ainsi s'agir d'un polydiméthylsiloxane sur lequel sont greffés, par l'intermédiaire d'un chaînon de raccordement de type thiopropylène, des motifs polymères mixtes du type acide poly(méth)acrylique et du type poly(méth) acrylate d'alkyle.

Un exemple particulièrement préféré de polymère siliconé greffé est le polysilicone-8 (connu sous l'abréviation CTFA) qui est un polydiméthylsiloxane sur lequel sont greffés, par l'intermédiaire d'un chaînon de raccordement de type thiopropylène, des motifs polymères mixtes du type acide poly(méth)acrylique et du type poly(méth)acrylate d'alkyle. Un polymère de ce type est notamment disponible sous la dénomination commerciale VS 80 (à 10% dans l'eau) ou LO 21 (sous forme pulvérulente) auprès de la société 3M. Il s'agit d'un copolymère de polydiméthylsiloxane à groupements

propylthio, d'acrylate de méthyle, de méthacrylate de méthyle et d'acide méthacrylique.

Les polymères synthétiques susmentionnés peuvent se présenter sous forme de latex.

Comme latex approprié pouvant être utilisé selon l'invention en tant qu'agent tenseur, on peut citer notamment les dispersions de polyesters-polyuréthanne et de polyéther-polyuréthanne telles que celles commercialisées sous les dénominations « Avalure UR410 et UR460 » par la société Noveon, et sous les dénominations « Neorez R974 », « Neorez R981 », « Neorez R970 », ainsi que les dispersions de copolymère acrylique telles que celles commercialisées sous la dénomination « Neocryl XK-90 » par la société Avecia.

On pourra aussi utiliser selon l'invention des polymères hydrosolubles ou hydrodispersibles comprenant des unités hydrosolubles ou hydrodispersibles et comprenant des unités à LSCT, lesdites unités à LCST présentant, en particulier, une température de démixtion dans l'eau de 5 à 40°C à une concentration massique de 1%. Ce type de polymère est plus amplement décrit dans la demande de brevet FR 2 819 429 [6].

D'autres polymères synthétiques pouvant être utilisés en tant qu'agent tenseur dans le cadre de l'invention sont les polymères éthyléniques séquencés linéaires filmogènes non élastomères et non hydrosolubles présentant un module dynamique de conservation E' à 1Hz et à 22°C supérieur à 200 MPa, tels que ceux décrits dans la demande FR 03 11346 [7].

Enfin, des agents tenseurs pouvant être utilisés dans le cadre de l'invention peuvent être une dispersion dans une phase grasse liquide de particules solides d'un polymère éthylénique greffé présentant une température de transition vitreuse supérieure à 40°C. Il est entendu que cette dispersion telle que définie dans le paragraphe ci-dessus diffère de celle incorporée dans la composition en vue de renforcer la rémanence de l'effet tenseur, cette dernière ayant, avantageusement, dans ce cas une température de transition vitreuse inférieure ou égale à 40°C. On peut citer, à titre d'exemple, d'agent tenseur de ce type, une dispersion obtenue par polymérisation dans l'isododécane d'acrylate de méthyle, d'acide acrylique et du macromonomère méthacrylate de polyéthylène/polybutylène (tel que le Kraton L-1253).

b) Les polymères d'origine naturelle.

Les polymères d'origine naturelle utilisables en tant qu'agent tenseur peuvent être choisis parmi :

- les protéines végétales et hydrolysats de protéines végétales ;
- les polysaccharides d'origine végétale sous forme de microgels, tels que l'amidon ;
- les latex d'origine végétale,
- et les mélanges de ceux-ci.

Des exemples de protéines végétales et hydrolysats de protéines végétales utilisables comme agents tenseurs selon l'invention sont constitués des protéines et hydrolysats de protéines de maïs, de seigle, de froment, de sarrasin, de sésame, d'épeautre, de pois, de fève, de lentille, de soja et de lupin.

Des polysaccharides convenant pour la formulation des compositions selon l'invention sont tous des polysaccharides d'origine naturelle, capables de former des gels thermoréversibles ou réticulés ainsi que des solutions. On entend par thermoréversible le fait que l'état gel de ces solutions de polymère est obtenu de façon réversible une fois la solution refroidie en dessous de la température de gélification caractéristique du polysaccharide utilisé.

Une première famille de polysaccharides d'origine naturelle qui peut être utilisée dans la présente invention est constituée par les carraghénanes et tout particulièrement le kappa-carraghénane et le iota-carraghénane. Ce sont des polysaccharides linéaires présents dans certaines algues rouges. Ils sont constitués de résidus $\beta$-1,3 et $\alpha$-1,4 galactoses en alternance, de nombreux résidus galactoses pouvant être sulfatés. Cette famille de polysaccharides est décrite dans le livre « Food Gels » édité par Peter HARRIS, Elsevier 1989, chap.3 [8]. Une autre famille de polysaccharides qui peut être utilisée est constituée par les Agars. Ce sont également des polymères extraits d'algues rouges et ils sont constitués de résidus 1,4-L-galactose et 1,3-D-galactose en alternance. Cette famille de polysaccharides est également décrite dans le chapitre 1 du livre « Food Gels » [9] mentionné précédemment. Une troisième famille de polysaccharides est constituée par des polysaccharides d'origine bactérienne appelés gellanes. Ce sont des polysaccharides constitués d'une alternance de résidus glucose, acide glucoronique et rhamnose. Ces gellanes sont décrits en particulier au chapitre 6 du livre « Food Gels » [10] mentionné précédemment. Dans le cas des polysaccharides formant des gels de type réticulé, en particulier induits par ajout de sels, on citera les polysaccharides appartenant à la famille des alginates et des pectines.

On peut aussi citer les pullulanes et leurs dérivés, ainsi que les mélanges de polymères de charges opposés qui par le biais d'interactions électrostatiques forment des complexes.

Les polysaccharides tenseurs sont présents sous forme de microgels tel que décrit dans FR 2 829 025 [11].

Une catégorie de polysaccharides utilisables selon l'invention particulièrement intéressante est constituée par l'amidon et ses dérivés.

L'amidon est un produit naturel bien connu de l'homme du métier. Il consiste en un polymère ou un mélange

de polymères, linéaires ou branchés, constitués d'unités d'α-D-glucopyranosyle. L'amidon est décrit en particulier dans « KIRK-OTHMER ENCYCLOPEDIA OF CHEMICAL TECHNOLOGY, 3ème édition, volume 21, p.492-507, Wiley Interscience, 1983 » [12].

L'amidon employé selon la présente invention peut être de toute origine : riz, maïs, pomme de terre, manioc, pois, froment, avoine, etc. Il peut être naturel ou éventuellement modifié par un traitement de type réticulation, acétylation, oxydation. Il peut être éventuellement greffé.

c) Les silicates mixtes.

Une autre classe d'agents tenseurs utilisables selon l'invention est constituée par les silicates mixtes. Par cette expression, on entend tous les silicates d'origine naturelle ou synthétique renfermant plusieurs types de cations choisis parmi les métaux alcalins (par exemple Na, Li, K) ou alcalino-terreux (par exemple Be, Mg, Ca) et les métaux de transition.

On utilise de préférence des phyllosilicates, à savoir des silicates ayant une structure dans laquelle les tétraèdres $SiO_4$ sont organisés en feuillets entre lesquels se trouvent enfermés les cations métalliques.

Une famille de silicates particulièrement préférée comme agents tenseurs est celle des laponites. Les laponites sont des silicates de magnésium, de lithium et de sodium ayant une structure en couches semblable à celle des montmorillonites. La laponite est la forme synthétique du minéral naturel appelé "hectorite". On peut utiliser par exemple la laponite commercialisée sous la dénomination Laponite XLS ou Laponite XLG par la société ROCKWOOD.

d) Les microdispersions de cire.

Une autre classe encore d'agents tenseurs utilisables dans la présente invention est constituée par les microdispersions de particules de cire. Il s'agit de dispersions de particules ayant un diamètre généralement inférieur à 5 μm, ou mieux, à 0,5 μm, et constituées essentiellement d'une cire ou d'un mélange de cires choisies par exemple parmi les cires de Carnauba, de Candelila ou d'Alfa. Le point de fusion de la cire ou du mélange de cires est de préférence compris entre 50°C et 150°C.

e) Les particules colloïdales de charges inorganiques.

En variante encore, on peut utiliser comme agent tenseur selon l'invention des particules colloïdales de charges inorganiques. Par "particules colloïdales", on entend des particules colloïdales en dispersion dans un milieu aqueux, hydroalcoolique, alcoolique ayant un diamètre moyen en nombre compris entre 0,1 et 100 nm, de préférence entre 3 et 30 nm.

Ces particules se présentent sous la forme de dispersions aqueuses et n'ont aucune propriété épaississante dans l'eau, l'alcool, l'huile et tous autres solvants. A une concentration supérieure ou égale à 15% en poids dans l'eau, la viscosité des solutions ainsi obtenues est inférieure à 0,05 Pa.s pour un taux de cisaillement égale à 10 $s^{-1}$. Les mesures de viscosité sont réalisées à 25°C à l'aide d'un rhéomètre Rheostress RS150 de Haake en configuration cône-plan, les dimensions du cône de mesure étant : Diamètre :60 mm et angle :2°.

Des exemples de charges inorganiques comprennent : la silice, l'oxyde de cérium, l'oxyde de zirconium, l'alumine, le carbonate de calcium, le sulfate de baryum, le sulfate de calcium, l'oxyde de zinc et le dioxyde de titane. Une charge inorganique particulièrement préférée est la silice. Des particules colloïdales de silice sont notamment disponibles sous forme de dispersion aqueuse de silice colloïdale auprès de la société CATALYSTS & CHEMICALS sous les dénominations commerciales COSMO S-40 et COSMO S-50.

Un exemple particulier de particules colloïdales de charges minérales peut être les particules colloïdales composites silice-alumine. Par composite silice-alumine, on entend des particules de silice dans lesquelles les atomes d'aluminium ont été substitués en partie par des atomes de silice.

A un pH 7, les particules colloïdales composites silice-alumine selon l'invention ont un potentiel zêta inférieur à -20 mV et de préférence inférieur à -25 mV. Les mesures sont réalisées à 25°C à l'aide d'un appareil DELSA 440SX de COULTER Scientific Instrument.

Comme particules colloïdales composites silice-alumine utilisables dans les compositions selon l'invention, on peut citer par exemple celles commercialisées par la société Grace sous les noms de Ludox AM, Ludox HSA et Ludox TMA.

Quelle que soit la nature de l'agent tenseur présent dans la composition, celui-ci est présent dans la composition à une teneur allant de 0,1 à 20% en poids du poids total de la composition, de préférence de 1 à 10%.

Comme mentionné précédemment, la composition comprend une dispersion de particules solides, dans une phase grasse liquide, d'un polymère éthylénique greffé.

Pour constituer une telle dispersion de particules solides, les polymères résultent d'un choix judicieux de monomères constituant la chaîne principale (par exemple, constituée majoritairement de monomères acrylates ou méthacrylates

d'alkyle court) et de macromonomères constituant les greffons (par exemple présents en une proportion représentant moins de 20% du poids du polymère).
Une telle dispersion en association avec un agent tenseur confère à la composition, dans laquelle elle est incorporée, un effet tenseur rémanent, grâce à sa capacité à renforcer le film tenseur tout en lui apportant des propriétés de souplesse. Cette rémanence est quantifiée selon l'invention notamment par la mesure de l'amélioration des propriétés mécaniques du film tenseur (plus particulièrement par la mesure de l'amélioration de la résistance à la rupture), comme cela sera explicité dans le protocole figurant dans la partie expérimentale de cette description.
Cette dispersion est présente, avantageusement, dans la composition à une teneur allant, en matière active, de 0,01 à 20% du poids total de la composition, de préférence de 1 à 10%, ladite dispersion étant présente, de préférence, au plus en une quantité égale à celle de l'agent tenseur.

Selon un mode particulier de l'invention, le polymère éthylénique greffé formant une dispersion de particules solides dans une phase grasse liquide (et remplissant le rôle d'agent renforçateur de l'effet tenseur) comprend un squelette insoluble dans ladite phase grasse liquide, et une partie soluble dans ladite phase grasse liquide constituée de chaînes latérales liées de manière covalente audit squelette.

En particulier, le polymère éthylénique greffé peut être un polymère acrylique greffé.

Un tel polymère acrylique greffé peut être notamment susceptible d'être obtenu par polymérisation radicalaire dans ladite phase grasse liquide:

- d'au moins un monomère acrylique, et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 et la teneur en macromonomère polymérisé représentant de 0,05 à 20 % en poids du polymère.

La masse moléculaire moyenne en poids du polymère peut aller, selon l'invention, de 10 000 à 300 000, de préférence de 20 000 à 200 000, et mieux encore de 25 000 à 150 000.

Le choix des monomères constituant le squelette du polymère et des macromonomères, de même que la masse moléculaire moyenne en poids du polymère, des chaînes latérales ainsi que la proportion des chaînes latérales pourra être fait en fonction de la phase grasse liquide choisie de manière à obtenir une dispersion de particules solides de polymères dans ladite phase, et de manière avantageuse une dispersion stable, ce choix pouvant être effectué par l'homme du métier.

Par "dispersion stable", on entend une dispersion qui n'est pas susceptible de former de dépôt solide ou de déphasage liquide/solide notamment après une centrifugation, par exemple, à 4000 tours/minute pendant 15 minutes.

Le polymère acrylique greffé peut être un polymère statistique.

Ainsi, le polymère acrylique greffé comprend un squelette (ou chaîne principale) constitué par un enchaînement de motifs acryliques résultant de la polymérisation notamment d'un ou plusieurs monomères acryliques et des chaînes latérales (ou greffons) issus de la réaction des macromonomères, lesdites chaînes latérales étant liées de manière covalente à ladite chaîne principale.

Le squelette (ou chaîne principale) est insoluble dans la phase grasse liquide considérée alors que les chaînes latérales (ou greffons) sont solubles dans ladite phase.

Grâce aux caractéristiques susmentionnées, dans une phase liquide donnée, les polymères ont la capacité de se replier sur eux-mêmes, formant ainsi des particules de forme sensiblement sphérique, avec sur le pourtour de ces particules les chaînes latérales déployées, qui assurent la stabilité de ces particules. De telles particules résultant des caractéristiques du polymère greffé ont la particularité de ne pas s'agglomérer dans ladite phase et donc de s'autostabiliser et de former une dispersion de particules de polymère particulièrement stable.

En particulier, les polymères de l'invention peuvent former des particules nanométriques, de taille moyenne allant de 10 à 400 nm, de préférence de 20 à 200 nm dans la phase grasse liquide considérée.

Du fait de cette taille très faible, les particules de polymère greffé en dispersion sont particulièrement stables et donc peu susceptibles de former des agglomérats.

La dispersion de polymère greffé peut donc être une dispersion stable dans la phase considérée et ne forme pas de sédiments, lorsqu'elle est placée pendant une durée prolongée (par exemple 24 heures) à température ambiante (25 °C).

Les tailles de particules peuvent être mesurées par différentes techniques. On peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.

De préférence, les tailles et les distributions de tailles de particules des compositions selon l'invention, sont mesurées par diffusion statique de la lumière au moyen d'un granulomètre commercial de type MasterSizer 2000 de

chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., « Light Scattering by Small Particles », Chapitres 9 et 10, Wiley, New York, 1957 [13].

La composition est caractérisée par son diamètre « effectif » moyen en volume D[4,3], défini de la manière suivante :

$$D[4,3] = \frac{\sum_i V_i \cdot d_i}{\sum_i V_i}$$

où Vi représente le volume des particules de diamètre effectif d. Ce paramètre est notamment décrit dans la documentation technique du granulomètre.

Les mesures sont réalisées à 25°C, sur une dispersion de particules diluée, obtenue à partir de la composition de la manière suivante :

1)dilution d'un facteur 100 avec de l'eau,
2) homogénéisation de la solution,
3) repos de la solution durant 18 heures,
4) récupération du surnageant homogène blanchâtre.

Le diamètre « effectif » est obtenu en prenant un indice de réfraction de 1,33 pour l'eau et un indice de réfraction moyen de 1,42 pour les particules.

Avantageusement, les monomères acryliques représentent de 50 à 100 % en poids, de préférence de 55 à 100 % en poids (notamment de 55 à 95 % en poids), préférentiellement de 60 à 100 % en poids (notamment de 60 à 90 % en poids) du mélange monomères acryliques + monomères vinyliques non acryliques éventuels.

De préférence, les monomères acryliques sont choisis parmi les monomères dont l'homopolymère est insoluble dans la phase grasse liquide considérée, c'est-à-dire que l'homopolymère est sous forme solide (ou non dissous) à une concentration supérieure ou égale à 5% en poids à température ambiante (20°C) dans ladite phase grasse liquide, ledit % étant exprimé par rapport au poids total du mélange constitué par l'homopolymère et la phase grasse liquide considérée.

Par "monomère acrylique", on entend dans la présente demande des monomères choisis parmi l'acide (méth) acrylique, les esters de l'acide (méth)acrylique (appelés également les (méth)acrylates), les amides de l'acide (méth) acrylique (appelés également les (méth)acrylamides).

Comme monomère acrylique susceptible d'être employé pour former le squelette insoluble du polymère, on peut citer, seul ou en mélange, les monomères suivants :

- (i) les (méth)acrylates de formule (IV) suivante :

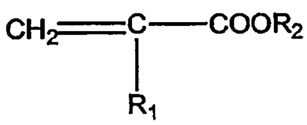

(IV)

dans laquelle :

- $R_1$ désigne un atome d'hydrogène ou un groupe méthyle ;
- $R_2$ représente un groupe choisi parmi :
  - un groupe alkyle, linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un

ou plusieurs substituants choisis parmi - OH, les atomes d'halogène (F, Cl, Br, I) et -NR'R'' avec R' et R'', identiques ou différents, étant choisis parmi les groupes alkyles linéaires ou ramifiés en $C_1$-$C_4$; et/ou pouvant être substitué par au moins un groupe polyoxyalkylène, en particulier un groupe alkylène en $C_1$-$C_4$, notamment un polyoxyéthylène et/ou un polyoxypropylène, ledit groupe polyoxyalkylène étant constitué par la répétition de 5 à 30 motifs oxyalkylène;

- un groupe alkyle cyclique comprenant de 3 à 6 atomes de carbone, ledit groupe pouvant comporter un ou plusieurs hétéroatomes choisis parmi O, N et S, et/ou pouvant comporter un ou plusieurs substituants choisis parmi OH et les atomes d'halogène (F, Cl, Br, I);

A titre d'exemples de $R_2$, on peut citer le groupe méthyle, éthyle, propyle, butyle, isobutyle, méthoxyéthyle, éthoxyéthyle, méthoxy-polyoxyéthylène 30 OE (OE signifiant oxyéthylène), trifluoroéthyle, 2-hydroxyéthyle, 2-hydroxypropyle, diméthylaminoéthyle, diéthylaminoéthyle, diméthylaminopropyle.

- (ii) les (méth)acrylamides de formule (V) suivante :

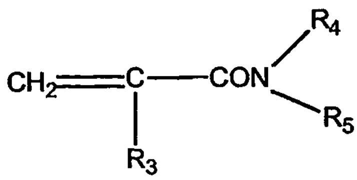

(V)

dans laquelle :

- $R_3$ désigne un atome d'hydrogène ou un groupe méthyle ;
- $R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi - OH, les atomes d'halogène (F, Cl, Br, I) et -NR'R" avec R' et R", identiques ou différents, étant choisis parmi les groupes alkyles linéaires ou ramifiés en $C_1$-$C_4$; ou
- $R_4$ représente un atome d'hydrogène et $R_5$ représente un groupe 1,1-diméthyl-3-oxobutyle.

A titre d'exemples de groupes alkyles pouvant constituer $R_4$ et $R_5$, on peut citer les groupes n-butyle, t-butyle, n-propyle, diméthylaminoéthyle, diéthylaminoéthyle, diméthylaminopropyle.

- (iii) les monomères (méth)acryliques comprenant au moins une fonction acide carboxylique, phosphorique ou sulfonique, tels que l'acide acrylique, l'acide méthacrylique, l'acide acrylamidométhylpropanesulfonique ;

lesdits monomères pouvant être présents sous forme de sels.

Parmi ces monomères acryliques, on peut tout particulièrement citer les (méth)acrylates de méthyle, d'éthyle, de propyle, de butyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle ; le diméthylaminopropylméthacrylamide; l'acide (méth)acrylique et leurs sels.

De préférence, les monomères acryliques sont choisis parmi l'acrylate de méthyle, l'acrylate de méthoxyéthyle, le méthacrylate de méthyle, le méthacrylate de 2-hydroxyéthyle, l'acide (méth)acrylique, le méthacrylate de diméthylaminoéthyle, et leurs mélanges.

Parmi les monomères additionnels vinyliques non acryliques, on peut citer :

- les esters vinyliques de formule (VI) suivante :

$$R_6\text{-COO-CH=CH}_2 \qquad \text{(VI)}$$

dans laquelle $R_6$ représente un groupe alkyle, linéaire ou ramifié, comprenant de 1 à 6 atomes, ou un groupe alkyle cyclique comportant de 3 à 6 atomes de carbone et/ou un groupe aromatique, par exemple de type benzénique,

anthracénique, et naphtalénique ;

- les monomères vinyliques non acryliques comprenant au moins une fonction acide carboxylique, phosphorique ou sulfonique, tels que l'acide crotonique, l'anhydride maléique, l'acide itaconique, l'acide fumarique, l'acide maléique, l'acide styrènesulfonique, l'acide vinylbenzoïque, l'acide vinylphosphorique et les sels de ceux-ci ;
- les monomères vinyliques non acryliques comprenant au moins une fonction amine tertiaire, tels que la 2-vinylpyridine, la 4-vinylpyridine ;
- et leurs mélanges.

Parmi les sels, on peut citer ceux obtenus par neutralisation des groupements acides à l'aide de base inorganiques telles que l'hydroxyde de sodium, l'hydroxyde de potassium, l'hydroxyde d'ammonium ou de bases organiques de type alcanolamines comme la monoéthanolamine, la diéthanolamine, la triéthanolamine, la 2-méthyl-2-amino-1-propanol.

On peut également citer les sels formés par neutralisation, le cas échéant, des motifs amine tertiaire, par exemple à l'aide d'un acide minéral ou organique. Parmi les acides minéraux, on peut citer l'acide sulfurique ou l'acide chlorhydrique, l'acide bromhydrique, iodhydrique, l'acide phosphorique, l'acide borique. Parmi les acides organiques, on peut citer les acides comportant un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique ou l'acide propionique, l'acide téréphtalique, ainsi que l'acide citrique et l'acide tartrique.

Le polymère greffé peut ne pas contenir de monomères vinyliques non acryliques additionnels tels que décrits précédemment. Dans ce mode de réalisation, le squelette insoluble du polymère greffé est formé uniquement de monomères acryliques tels que décrits précédemment.

Il est entendu que ces monomères acryliques non polymérisés peuvent être solubles dans le milieu considéré, mais le polymère formé par polymérisation de ces monomères est insoluble dans la phase grasse liquide considérée.

Selon l'invention, le polymère acrylique greffé comprend des chaînes latérales issues de la polymérisation de macromonomères ayant un groupe terminal polymérisable.

On entend par "macromonomère ayant un groupe terminal polymérisable" tout oligomère comportant sur une seule de ses extrémités un groupe terminal polymérisable apte à réagir lors de la réaction de polymérisation avec les monomères acryliques et éventuellement les monomères vinyliques non acryliques additionnels constituant le squelette. Le macromonomère permet de former les chaînes latérales du polymère acrylique greffé. Le groupe polymérisable du macromonomère peut être avantageusement un groupe à insaturation éthylénique susceptible de se polymériser par voie radicalaire avec les monomères constituant le squelette.

Les macromonomères comportent à une des extrémités de la chaîne un groupe terminal polymérisable apte à réagir au cours de la polymérisation avec les monomères acryliques et éventuellement les monomères vinyliques additionnels, pour former les chaînes latérales du polymère greffé. Ledit groupe terminal polymérisable peut être en particulier un groupe vinyle ou (méth)acrylate (ou (méth)acryloxy), et de préférence un groupe (méth)acrylate.

Les macromonomères sont choisis préférentiellement parmi les macromonomères dont l'homopolymère a une température de transition vitreuse (Tg) inférieure ou égale à 25°C, notamment allant de - 100°C à 25°C, de préférence allant de -80°C à 0°C.

De préférence, le macromonomère est choisi parmi les macromonomères dont l'homopolymère est soluble dans la phase grasse liquide considérée, c'est-à-dire complètement dissous à une concentration supérieure ou égale à 5 % en poids et à température ambiante (20°C) dans ladite phase grasse liquide, ledit % étant exprimé par rapport au poids total du mélange constitué par l'homopolymère et de la phase grasse liquide considérée.

De préférence, le macromonomère polymérisé (constituant les chaînes latérales du polymère greffé) représente de 0,1 à 15 % en poids du poids total du polymère, préférentiellement de 0,2 à 10 % en poids, et plus préférentiellement de 0,3 à 8 % en poids.

Les macromonomères ont, généralement, une masse moléculaire moyenne en poids (Mw) supérieure à 200, de préférence à 300, mieux, à 500 et, encore mieux, à 600. Ils ont, de préférence, une masse moléculaire moyenne en poids allant de 200 à 100 000, de préférence allant de 500 à 50 000, préférentiellement allant de 800 à 20 000, plus préférentiellement allant de 800 à 10000, et encore plus préférentiellement allant de 800 à 6000.

Dans la présente demande, les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).

Comme énoncé précédemment, les agents renforçateurs de l'invention constituent une dispersion de particules solides d'un polymère éthylénique greffé dans une phase grasse liquide.

Cette phase grasse liquide est constituée principalement d'un ou plusieurs composés organiques liquides qui seront définis ci-après.

On entend par "composé organique liquide" un composé non aqueux qui est à l'état liquide à la température ambiante (25 °C), c'est-à-dire qui s'écoule sous l'effet de son propre poids.
Parmi les composés organiques liquides pouvant être présents dans la phase grasse liquide, on peut citer :

- les composés organiques liquides ayant un paramètre de solubilité global selon l'espace de solubilité de Hansen inférieur ou égal à 18 $(MPa)^{½}$, de préférence, inférieur ou égal à 17 $(MPa)^{½}$,
- les monoalcools ayant un paramètre de solubilité global selon l'espace de solubilité de Hansen inférieur ou égal à 20 $(MPa)^{½}$ ; et
- leurs mélanges.

Le paramètre de solubilité global δ selon l'espace de solubilité de Hansen est défini dans l'article « Solubility parameter values » de Eric A.Grulke de l'ouvrage « Polymer Handbook », 3éme édition, Chapitre VII, p.519-559 [14] par la relation :

$$\delta = (d_D{}^2 + d_P{}^2 + d_H{}^2)^{1/2}$$

dans laquelle

- $d_D$ caractérise les forces de dispersion de LONDON issues de la formation de dipôles induits lors des chocs moléculaires,
- $d_P$ caractérise les forces d'interactions de DEBYE entre dipôles permanents, et
- $d_H$ caractérise les forces d'interactions spécifiques (type liaisons hydrogène, acide/base, donneur/accepteur, etc.).

La définition des solvants dans l'espace de solubilité selon Hansen est décrite dans l'article de C.M.Hansen « The three dimensional solubility parameters » J.Paint Technol. 39, 105 (1967) [15].
Selon une première alternative, la phase grasse liquide peut être une phase grasse non siliconée.
On entend par "phase grasse liquide non siliconée" une phase grasse comprenant un ou plusieurs composés organiques liquides non siliconé(e)s choisis parmi :

- les composés liquides organiques non siliconés ayant un paramètre de solubilité global selon l'espace de solubilité de Hansen inférieur ou égal à 18 $(MPa)^{½}$, de préférence inférieur ou égal à 17 $(MPa)^{½}$ ;
- les monoalcools liquides ayant un paramètre de solubilité global selon l'espace de solubilité de Hansen inférieur ou égal à 20 $(MPa)^{½}$; et
- leurs mélanges,

lesdits composés non siliconés étant présents majoritairement dans la phase grasse liquide, c'est-à-dire à au moins 50 % en poids, notamment de 50 à 100 % en poids, par exemple de 60 à 99 % en poids, ou encore de 65 à 95 % en poids, par rapport au poids total de la phase grasse liquide.
Ladite phase grasse liquide non siliconée peut donc éventuellement comprendre des composés organiques liquides ou huiles siliconé(e)s, tels que ceux cités ci-après, qui peuvent être présents en une quantité inférieure à 50 % en poids, notamment allant de 0,1 à 40 % en poids, voire allant de 1 à 35 % en poids, ou encore allant de 5 à 30 % en poids, par rapport au poids total de la phase grasse liquide.
Selon un mode particulier de réalisation de l'invention, la phase grasse liquide non siliconée ne contient pas de composés organiques liquides ou huiles siliconé(e)s.
Les composés liquides non siliconés ayant un paramètre de solubilité global selon l'espace de solubilité de Hansen inférieur à 18 $(MPa)^{½}$, de préférence inférieur ou égal à 17 $(MPa)^{½}$ peuvent être choisis parmi :

- les huiles naturelles ou synthétiques, carbonées, hydrocarbonées ou fluorées, éventuellement ramifiées, seules ou en mélange ;
- les alcanes linéaires, ramifiées et/ou cycliques, éventuellement volatils ;
- les esters et notamment les esters linéaires, ramifiés ou cycliques, ayant au moins 6 atomes de carbone, notamment ayant de 6 à 30 atomes de carbone ;
- les éthers et notamment les éthers ayant au moins 6 atomes de carbone, notamment ayant de 6 à 30 atomes de carbone ;
- les cétones et notamment les cétones ayant au moins 6 atomes de carbone, notamment ayant de 6 à 30 atomes de carbone.

Parmi les huiles, on peut citer les huiles végétales formées par des esters d'acides gras et de polyols, en

particulier les triglycérides, telles que l'huile de tournesol, de sésame ou de colza.

On peut également citer les alcanes linéaires, ramifiés et/ou cycliques éventuellement volatils et notamment des huiles de paraffine, de vaseline, ou le polyisobutylène hydrogéné, l'isododécane, ou encore les 'ISOPARS', les isoparaffines volatiles.

Par monoalcools liquides ayant un paramètre de solubilité global selon l'espace de solubilité de Hansen inférieur ou égal à 20 $(MPa)^{1/2}$, on entend des monoalcools liquides gras aliphatiques, saturés ou insaturés, ayant au moins 6 atomes de carbone, par exemple de 6 à 30 atomes de carbone, la chaîne hydrocarbonée ne comportant pas de groupement de substitution. Comme monoalcools selon l'invention, on peut citer l'alcool oléique, le décanol et l'alcool linoléique.

Dans une telle phase grasse liquide, les polymères formant dispersion peuvent selon un mode de réalisation de l'invention être des polymères acryliques greffés tels que définis précédemment.

De préférence, les macromonomères présents dans un tel polymère greffé sont avantageusement des macromonomères carbonés.

Par "macromonomère carboné" on entend un macromonomère non siliconé, et notamment un macromonomère oligomère obtenu par polymérisation de monomère(s) non siliconé(s) à insaturation éthylénique, et principalement par polymérisation de monomères acryliques et/ou vinyliques non acryliques.

Comme macromonomères carbonés, on peut en particulier citer :

- (i) les homopolymères et les copolymères (méth)acrylate d'alkyle linéaire ou ramifié en C8-C22, présentant un groupe terminal polymérisable choisi parmi les groupes vinyle ou (méth)acrylate parmi lesquels on peut citer en particulier : les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate ; les macromonomères de poly(acrylate de stéaryle) ou de poly (méthacrylate de stéaryle) à extrémité mono(méth) acrylate.
  De tels macromonomères sont notamment décrits dans les brevets EP 895467. [16] et EP 96459 [17] et dans l'article Gillman K.F., Polymer Letters, Vol 5, page 477-481 (1967) [18].
  On peut en particulier citer les macromonomères à base de poly(acrylate d'éthyl-2-hexyle) ou de poly(acrylate de dodécyle) à extrémité mono(méth)acrylate.
- (ii) les polyoléfines ayant un groupe terminal à insaturation éthylénique, en particulier ayant un groupement terminal (méth)acrylate. Comme exemple de telles polyoléfines, on peut citer en particulier les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène; les macromonomères de polyisoprène ; les macromonomères de polybutadiène; les macromonomères de poly(éthylène/butylène)-polyisoprène ;

De tels macromonomères sont en particulier décrits dans US 5,625,005 [19] qui mentionne des macromonomères polyéthylène/polybutylène et polyéthylène/polypropylène à groupement terminal réactif (méth)acrylate.

On peut en particulier citer le méthacrylate de poly(éthylène/butylène), tel que celui commercialisé sous la dénomination Kraton Liquid L-1253 par Kraton Polymers.

Une composition particulièrement efficace, dans le cadre de l'invention, est une composition dans laquelle la dispersion (remplissant le rôle d'agent renforçateur) est une dispersion obtenue par polymérisation de l'acrylate de méthyle et du macromonomère méthacrylate de polyéthylène/polybutylène (notamment Kraton L-1253) dans l'isododécane et l'agent tenseur est, de préférence, une dispersion colloïdale de silice.

Une autre composition particulièrement efficace dans le cadre de l'invention est une composition dans laquelle la dispersion (remplissant le rôle d'agent renforçateur) est une dispersion obtenue par polymérisation de l'acrylate de méthyle et du macromonomère méthacrylate de polyéthylène/polybutylène (notamment Kraton L-1253) dans l'isododécane et l'agent tenseur est, de préférence, une dispersion par polymérisation dans l'isododécane d'acrylate de méthyle, d'acide acrylique et du macromonomère méthacrylate de polyéthylène/polybutylène (notamment Kraton L-1253).

Selon une seconde alternative, la phase grasse liquide peut être une phase grasse siliconée.

On entend par "phase grasse liquide siliconée" une phase grasse comprenant un ou plusieurs composés organiques liquides siliconés choisis parmi les composés liquides organiques siliconés ayant un paramètre de solubilité global selon l'espace de solubilité de Hansen inférieur ou égal à 17 $(MPa)^{1/2}$, lesdits composés siliconés étant présents majoritairement dans la phase grasse liquide, c'est-à-dire à au moins 50 % en poids, notamment de 50 à 100 % en poids, par exemple de 60 à 99 % en poids, ou encore de 65 à 95 % en poids, par rapport au poids total de la phase grasse liquide.

Ladite phase grasse liquide siliconée peut donc éventuellement comprendre des composés organiques liquides ou huiles non siliconé(e)s, tels que décrits précédemment, qui peuvent être présents en une quantité inférieure à 50 %

en poids, notamment allant de 0,1 à 40 % en poids, voire allant de 1 à 35 % en poids, ou encore allant de 5 à 30 % en poids, par rapport au poids total de la phase grasse liquide.

Selon un mode particulier de réalisation de l'invention, la phase grasse liquide siliconée ne contient pas de composés organiques liquides non siliconés.

Parmi les composés siliconés répondant à la définition ci-dessus, on peut citer les huiles siliconées telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines, et les huiles siliconées volatiles, notamment cycliques.

En particulier, on peut citer les huiles de silicone, éventuellement ramifiées, volatiles et/ou non volatiles.

Par huile volatile, on entend une huile susceptible de s'évaporer de la peau ou des lèvres en moins d'une heure, ayant notamment une pression de vapeur, à température ambiante et pression atmosphérique allant de $10^{-3}$ à 300 mm de Hg (0,13 Pa à 40 000 Pa).

Comme huile siliconée volatile utilisable dans l'invention, on peut citer les huiles siliconées linéaires ou cycliques ayant de 2 à 7 atomes de silicium, ces huiles siliconées comportant éventuellement des groupes alkyle ou alcoxy ayant de 1 à 10 atomes de carbone. En particulier, on peut citer l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme huile siliconée non volatile, on peut citer les polydialkylsiloxanes non volatils, tels que les polydiméthylsiloxanes (PDMS) non volatils; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényl triméthicones, les phényl diméthicones, les phényl triméthylsiloxy diphénylsiloxanes, les diphényl diméthicones, les diphényl méthyldiphényl trisiloxanes, les polyméthylphénylsiloxanes; les polysiloxanes modifiés par des acides gras (notamment en $C_8$-$C_{20}$), des alcools gras (notamment en $C_8$-$C_{20}$) ou des polyoxyalkylènes (notamment polyoxyéthylène et/ou polyoxypropylène); les polysiloxanes aminées ; les polysiloxanes à groupement hydroxyles; les polysiloxanes fluorés comportant un groupement fluoré pendant ou en bout de chaîne siliconée ayant de 1 à 12 atomes de carbone dont tout ou partie des hydrogène sont substitués par des atomes de fluor ; et leurs mélanges.

Dans une telle phase grasse liquide, les polymères formant une dispersion peuvent selon un mode de réalisation de l'invention être des polymères acryliques greffés tels que ceux définis précédemment.

De préférence, les macromonères présents dans un tel polymère acrylique greffé sont avantageusement des macromonomères siliconés.

Par "macromonomère siliconé", on entend un macromonomère organopolysiloxane, et en particulier un macromonomère polydiméthylsiloxane.

Des polymères formant dans une phase liquide siliconée une dispersion efficace en tant qu'agent renforçateur sont ceux choisis parmi les polymères susceptibles d'être obtenus par polymérisation radicalaire dans ladite phase:

- d'un monomère acrylique principal (c'est-à-dire représentant plus de 50% en poids) choisi parmi les (méth)acrylates d'alkyle en $C_1$-$C_3$, seul ou en mélange, et éventuellement d'un ou plusieurs monomères acryliques additionnels (c'est-à-dire représentant moins de 50% en poids) choisis parmi l'acide acrylique, l'acide méthacrylique et les (méth) acrylates d'alkyle de formule (VII) définie ci-après, et leurs sels, pour former ledit squelette insoluble ; et
- et d'au moins un macromonomère siliconé comportant un groupe terminal polymérisable tel que défini précédemment.

Comme monomère acrylique principal, on peut utiliser l'acrylate de méthyle, le méthacrylate de méthyle, l'acrylate d'éthyle, le méthacrylate d'éthyle, l'acrylate de n-propyle, le méthacrylate de n-propyle, l'acrylate d'iso-propyle et le méthacrylate d'isopropyle, et leurs mélanges.
On préfère tout particulièrement l'acrylate de méthyle, le méthacrylate de méthyle, le méthacrylate d'éthyle.

Les monomères acryliques additionnels peuvent être choisis parmi :

- l'acide (méth)acrylique et ses sels,
- les (méth)acrylates de formule (VII) et leurs sels :

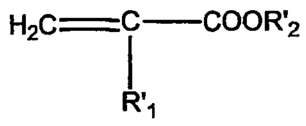

(VII)

dans laquelle :

- $R'_1$ désigne un atome d'hydrogène ou un groupe méthyle ;
- $R'_2$ représente :
  - un groupe alkyle, linéaire ou ramifié, comprenant de 1 à 6 atomes de carbone, ledit groupe comportant dans sa chaîne un ou plusieurs atomes d'oxygène et/ou comportant un ou plusieurs substituants choisis parmi -OH, les atomes d'halogène (F, Cl, Br, I) et -NR'R" avec R' et R", identiques ou différents, étant choisis parmi les groupes alkyles, linéaires ou ramifiés, en $C_1$-$C_3$ ; ou
  - un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I) ; ou
  - leurs mélanges.

A titre d'exemples de $R'_2$, on peut citer le groupe méthoxyéthyle, éthoxyéthyle, trifluoroéthyle; 2-hydroxyéthyle, 2-hydroxypropyle, diméthylaminoéthyle, diéthylaminoéthyle, diméthylaminopropyle.

Parmi ces monomères acryliques additionnels, on peut tout particulièrement citer l'acide (méth)acrylique, les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle, leurs sels, et leurs mélanges.
On préfère tout particulièrement l'acide acrylique, l'acide méthacrylique.

Comme macromonomères siliconés, on peut en particulier citer les polydiméthylsiloxanes à groupement terminal mono(méth)acrylate, et notamment ceux de formule (VIII) suivante :

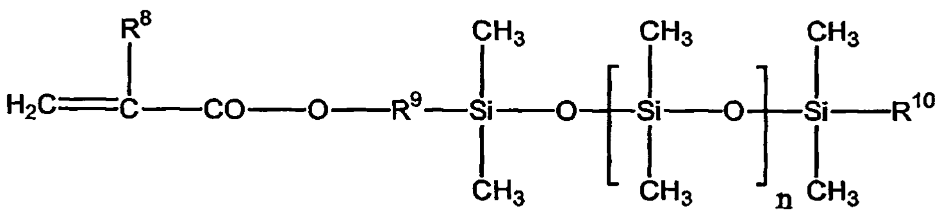

(VIII)

dans laquelle :

- $R^8$ représente un atome d'hydrogène ou un groupe méthyle ;
- $R^9$ représente un groupe hydrocarboné divalent ayant de 1 à 10 atomes de carbone et comprend éventuellement une ou deux liaisons éther -O- ;
- $R^{10}$ représente un groupe alkyle ayant de 1 à 10 atomes de carbone, notamment de 2 à 8 atomes de carbone ;
- n représente un nombre entier allant de 1 à 300, de préférence allant de 3 à 200, et préférentiellement allant de 5 à 100.

On peut en particulier citer les polydiméthylsiloxanes à groupement terminal mono (méth)acrylate, et notamment les monométhacryloxypropyl polydiméthylsiloxanes tels que ceux commercialisés sous la dénomination PS560-K6 par la société United Chemical Technologies Inc. (UCT) ou sous la dénomination MCR-M17 par la société Gelest Inc.

Une composition particulièrement efficace dans le cadre de l'invention est une composition dans laquelle la

dispersion (remplissant le rôle d'agent renforçateur) est une dispersion obtenue par polymérisation de l'acrylate de méthyle et du macromonomère monométhacryloxypropylpolydiméthylsiloxane dans le cyclopentadiméthylsiloxane et l'agent tenseur est, de préférence, une dispersion colloïdale de silice.

Une autre composition efficace dans le cadre de l'invention est une composition dans laquelle la dispersion (remplissant le rôle d'agent renforçateur) est une dispersion obtenue par polymérisation de l'acrylate de méthyle et du macromonomère monométhacryloxypropylpolydiméthylsiloxane dans le cyclopentadiméthylsiloxane et l'agent tenseur est un copolymère comprenant un squelette renfermant des unités (méth)acrylate d'isobutyle ou de tertiobutyle greffé par des greffons polydiméthylsiloxanes.

On peut préparer la dispersion de particules de polymère greffé par un procédé comprenant une étape de copolymérisation radicalaire, dans une phase grasse liquide répondant à la définition donnée précédemment, d'un ou plusieurs monomères acryliques (et éventuellement d'un ou plusieurs monomères additionnels vinyliques non acryliques) tels que définis précédemment avec un ou plusieurs macromonomères tels que définis précédemment.

D'une manière classique, la copolymérisation peut être effectuée en présence d'un initiateur de polymérisation. Les initiateurs de polymérisation peuvent être des amorceurs radicalaires. De manière générale, un tel initiateur de polymérisation peut être choisi parmi les composés organiques peroxydés tels que le dilauroyl peroxyde, le dibenzoyl peroxyde, le tert-butyl peroxy-2-éthylhexanoate ; les composés diazotés tels que l'azobisisobutyronitrile, l'azobisdiméthylvaleronitrile.

La réaction peut être également initiée à l'aide de photoinitiateurs ou par une radiation telle que des UV, des neutrons ou par plasma.

D'une manière générale, pour mettre en oeuvre ce procédé, on introduit, dans un réacteur de taille appropriée à la quantité de polymère que l'on va réaliser, au moins une partie de la phase grasse liquide, une partie des monomères acryliques et/ou vinyliques additionnels, qui constituera, après polymérisation, le squelette insoluble, la totalité du macromonomère (qui constituera les chaînes latérales du polymère) et une partie de l'initiateur de polymérisation. A ce stade d'introduction, le milieu réactionnel forme un milieu relativement homogène.

Le milieu réactionnel est ensuite agité et chauffé jusqu'à une température pour obtenir une polymérisation des monomères et macromonomères. Après un certain temps, le milieu initialement homogène et limpide conduit à une dispersion d'aspect laiteux. On ajoute ensuite un mélange constitué de la partie restante de monomères et de l'initiateur de polymérisation. Après un temps adéquat pendant lequel le mélange est chauffé sous agitation, le milieu se stabilise sous forme d'une dispersion laiteuse, la dispersion comprenant des particules de polymères stabilisés dans la phase grasse liquide dans laquelle elles ont été créées, ladite stabilisation étant due à la présence, dans le polymère, de chaînes latérales solubles dans ledit milieu.

La dispersion de polymère peut être présente à raison de 3 à 95% en poids de matière active dans la composition, notamment de 4 à 90 % en poids, voire de 20 à 70 % en poids, par rapport au poids total de la composition.
Notons enfin que les polymères greffés constituant la dispersion, améliorant les propriétés mécaniques du film tenseur, présentent avantageusement une température de transition vitreuse inférieure ou égale à 40°C.

Selon l'invention, la dispersion de polymères décrite ci-dessus est utilisée dans une composition cosmétique.

La composition selon l'invention comprend, comme cela a été mentionné ci-dessus, une phase grasse dans laquelle le polymère décrit ci-dessus forme une dispersion.

Ladite phase grasse représente, par exemple, de 0,5 à 80% du poids total de la composition, de préférence de 1 à 55% et mieux encore de 1 à 25%.

La composition comprend également avantageusement une phase aqueuse dans laquelle se trouve généralement l'agent tenseur, bien qu'il puisse en variante se trouver dans la phase grasse selon sa nature.

Ainsi, la composition de la présente invention peut être une émulsion, notamment une émulsion huile-dans-eau (H/E) ou eau dans huile (E/H) ou multiple (E/H/E ou polyol/H/E ou H/E/H), sous forme de crème, de gel, de lait, de pâte, de mousse, de lotion biphase ou multiphase.

Il est entendu que ladite composition cosmétique comprendra en outre le ou les polymère(s) susmentionné(s), un milieu physiologiquement acceptable adapté à une application topique sur la peau du visage.

Ledit milieu physiologiquement acceptable est généralement cosmétiquement acceptable, c'est-à-dire qu'il présente une odeur, une couleur et un toucher agréables, compatibles avec une utilisation cosmétique, et ne génère pas d'inconforts (picotements, tiraillements, rougeurs) susceptibles de détourner l'utilisateur de son emploi.

La composition selon l'invention peut également contenir des ingrédients couramment utilisés en cosmétique, tels que des agents épaississants, des agents séquestrants, des parfums, des agents alcalinisants ou acidifiants, des conservateurs, des filtres solaires, des tensioactifs, des charges, des pigments et colorants, et leurs mélanges.

Elle peut également contenir des actifs anti-âge à effet complémentaire aux polymères définis précédemment, tels qu'au moins un composé choisi parmi les agents desquamants, les agents hydratants, les agents stimulant la prolifération et/ou la différenciation des kératinocytes, les agents stimulant la synthèse du collagène et/ou de l'élastine ou prévenant leur dégradation, les agents dépigmentants, les agents anti-glycation, les agents stimulant la synthèse de glycosaminoglycannes, les agents dermo-décontractants ou myorelaxants, les agents antioxydants et anti-radicalaires,

et leurs mélanges.

Bien entendu, l'homme du métier veillera à choisir ce ou ces éventuels composés complémentaires et/ou leur quantité, de manière telle que les propriétés avantageuses de la composition correspondante selon l'invention ne soient pas ou substantiellement pas altérées par l'adjonction envisagée.

L'application de la composition se fait, généralement, selon les techniques habituelles, par exemple par application de crèmes, de gels, de sérums, de lotions, sur la peau destinée à être traitée, en particulier la peau du contour de l'oeil. Dans le cadre de ce procédé, la composition peut être, par exemple, une composition de soin ou une composition de maquillage, en particulier de fond de teint.

La présente invention a trait, selon un second objet, à une composition cosmétique comprenant, dans un milieu physiologiquement acceptable adapté à une application topique sur la peau du visage :

- de 0,1 à 20% en poids d'au moins un agent tenseur, par rapport au poids total de la composition, ledit agent tenseur étant sous forme de particules colloïdales de charges inorganiques ; et
- au moins une dispersion dans une phase grasse liquide de particules solides d'un polymère éthylénique greffé.

La présente invention a trait, selon un troisième objet, à l'utilisation d'une dispersion de particules solides d'un polymère éthylénique tel que défini précédemment pour améliorer la rémanence de l'effet tenseur procuré par un agent tenseur, ledit agent tenseur étant tel que défini précédemment.

La rémanence de l'effet tenseur est quantifiée par des tests figurant dans la partie expérimentale de cette description.

Selon un quatrième objet, la présente invention a trait à l'utilisation d'une dispersion de particules solides d'un polymère éthylénique dans une phase grasse liquide, tel qu'une huile volatile telle que définie ci-dessus, ledit polymère étant tel que défini précédemment dans une composition cosmétique comprenant en tant qu'agent tenseur une dispersion aqueuse de particules colloïdales inorganiques, en particulier de silice, pour prévenir le blanchiment de la peau tout en assurant une amélioration de la rémanence de l'effet tenseur induit par l'agent tenseur.

L'invention va maintenant être décrite en référence aux exemples suivants donnés à titre illustratif et non limitatif.

## BREVE DESCRIPTION DES FIGURES

La figure unique illustre une courbe représentant la force F (en newtons, N) en fonction du déplacement d (en mm), ladite courbe étant utilisée pour quantifier la rémanence de l'effet tenseur des compositions de l'invention.

## EXPOSÉ DÉTAILLÉ DE MODES DE RÉALISATION PARTICULIERS

Différents polymères et formulations ont été préparés, incorporant des polymères tels que définis ci-dessus en association avec des agents tenseurs, et testés afin de mettre en évidence l'amélioration de la rémanence de l'effet tenseur induit par l'utilisation de tels polymères dans ces compositions.

Avant de procéder à l'exposé détaillé des préparations de polymères et de formulations, nous allons exposer deux protocoles permettant de quantifier la rémanence de l'effet tenseur induit par l'association polymère/agent tenseur susmentionné.

<u>*Protocoles de quantification de la rémanence de l'effet tenseur</u>

<u>**Premier Protocole.*</u>

<u>*Principe du test*</u>

Les propriétés de rémanence que nous recherchons sont atteintes grâce à l'introduction de composés jouant le rôle d'agents renforçateurs, ces composés étant les polymères éthyléniques greffés sous forme de dispersion tels que définis ci-dessus. Le potentiel renforçateur des composés que nous utilisons a été quantifié à partir de la mesure de la résistance à la rupture des matériaux (dans le cas présent une crème anti rides).

L'essai consiste à solliciter en compression jusqu'à rupture le matériau déposé en surface d'une mousse souple et déformable. L'utilisation de ce support en mousse permet d'imposer une importante déformation au matériau déposé en surface, et donc une quantification de sa résistance à la rupture. La sollicitation mécanique en compression est exercée à l'aide d'un poinçon cylindrique de diamètre 1mm ; la vitesse de déplacement du poinçon étant de 0,1mm/s. L'essai est réalisé à l'aide d'un analyseur de texture TA-XT2i commercialisé par la société Stable Micro System. Il est ainsi obtenu une courbe de force F (en N) en fonction du déplacement d (en mm) à partir de laquelle il est possible de déterminer le point de rupture du matériau $F_{rupt}$(N) (conformément à ce qui est représenté sur la figure unique).

Deux paramètres sont retenus pour quantifier la résistance à la rupture du matériau :

① $F_{rupt}$ (N) : force à rupture
② $W_{rupt}$ ($J/m^2$) : énergie à rupture : surface sous la courbe Force=f(d) / surface du poinçon

Le substrat est constitué d'une mousse néoprène de 13 mm d'épaisseur. Le matériau (composition antirides de l'invention) est déposé sur ce substrat de façon à obtenir après un séchage de 24h un film d'une épaisseur de 15 à 30μm. Les dépôts ont été réalisés à l'aide d'un tireur de film déposant 650μm humide (c'est-à-dire avant séchage).

*Deuxième Protocole.*

Ce deuxième protocole vise à quantifier l'effet tenseur rémanent induit par les compositions de l'invention par un test *in vitro* de rétractation.

Plus précisément, ce protocole consiste à quantifier in *vitro* l'effet tenseur rémanent d'une composition déposée sur un substrat en élastomère ayant un module de l'ordre de 20 MPa et d'une épaisseur de 100 μm.

Dans un premier temps, une composition comprenant un agent tenseur à 20% en poids est déposée, en une quantité de 30 μL, sur le substrat sous forme d'éprouvette rectangulaire (10*40 mm) en élastomère. Après 3h de séchage à 22±3°C et à 40±10% d'humidité relative, la tension exercée par ce dépôt sur le substrat et par conséquent l'effet tenseur est directement relié à la diminution de la largeur au centre de l'éprouvette. Cet effet tenseur (ET) est alors quantifié de la manière suivante :

$$ET= (L_0-L_{3h}/L_0)*100 \text{ (exprimé en \%)}$$

avec :

- $L_0$ représentant la largeur initiale de l'éprouvette, c'est-à-dire 10 mmm ;
- $L_{3h}$ représentant la largeur de l'éprouvette après 3 heures de séchage.

Dans un deuxième temps, l'éprouvette est ensuite étirée manuellement de 50% (la longueur de l'éprouvette passant de 40 mm à 60 mm) et après retour à sa longueur initiale, sa largeur est de nouveau mesurée de manière à quantifier la rémanence de l'effet tenseur après sollicitation ($ET_r$).

La grandeur $ET_r$ est quantifiée de la manière suivante :

$$ET_r= (L_0-L_{100\%}/L_0)*100 \text{ (exprimé en \%)}$$

avec :

- Lo représentant la largeur initiale de l'éprouvette, c'est-à-dire 10 mmm ;
- $L_{100\%}$ représentant la largeur de l'éprouvette après que celle-ci ait subi la déformation définie ci-dessus et un retour à la longueur initiale.

Plus la grandeur $ET_r$ est élevée, plus la rémanence de l'effet tenseur est importante.

EXEMPLE COMPARATIF

Cet exemple illustre une composition cosmétique comprenant un agent tenseur sous forme d'une dispersion aqueuse de silice colloïdale (Cosmo S40), ladite composition étant dépourvue de polymère éthylénique greffé sous forme de dispersion conformément à la présente invention.

La composition est la suivante :

| Constituants | Quantité |
|---|---|
| Stéarate de glycéryle et Stéarate PEG-100 | 2 g |
| Tartrate de dimyristyle et alcool cétéarylique et $C_{12}$-$C_{15}$-pareth-7 et PPG-25-laureth-25 | 1,50 g |
| Cyclohexasiloxane | 10 g |
| Alcool stéarylique | 1 g |
| Eau | 66,75 g |
| Phénoxyéthanol | 1 g |
| Séquestrant | 0,05 g |
| Polyacrylamide (Hostacerin AMPS de Clariant) | 0,40 g |
| Gomme de xanthane | 0,20 g |
| Cosmo S40 (dispersion aqueuse de silice colloïdale) | 17,10 g |

La composition est préparée de la façon suivante :

La phase constituée de l'eau, du phénoxyéthanol, du séquestrant, et de la gomme de xanthane est chauffée à 75°C. On y incorpore ensuite le polymère épaississant (c'est-à-dire le polyacrylamide). Le mélange est agité jusqu'à obtention d'un gel homogène.

La phase constituée par le stéarate de glycéryle, le stéarate de PEG-100, le tartrate de dimyristyle, l'alcool cétéarylique, le $C_{12}$-$C_{15}$-pareth-7, le PPG-25-laureth-25, le cyclohexasiloxane et l'alcool stéarylique est chauffée à 75°C. Cette phase est ensuite incorporée à la phase précédente pour réaliser une émulsion. La dispersion aqueuse de silice colloïdale est ensuite incorporée dans l'émulsion à 40-45°C et l'agitation est maintenue jusqu'à refroidissement complet.

EXEMPLE 1

Cet exemple illustre la préparation et l'utilisation d'un polymère formant une dispersion de particules dans un solvant carboné, ledit polymère étant obtenu par polymérisation de l'acrylate de méthyle et du macromonomère méthacrylate de polyéthylène/polybutylène (Kraton L-1253) dans l'isododécane.

*Préparation de la dispersion

Dans un réacteur de 500 mL, on charge 50 g d'heptane, 50 g d'isododécane, 7g d'acrylate de méthyle et 3 g de macromonomère du type méthacrylate de polyéthylène/polybutylène (Kraton L-1253) et 0,8 g de tertio butyl peroxy-2-éthylhexanoate (Trigonox 21S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 40 g d'acrylate de méthyle et 0,5 g de Trigonox 21S.

On laisse ensuite le chauffage pendant 4 heures à 90°C puis on distille l'heptane du milieu réactionnel.

A l'issue de cette opération de distillation, on obtient une dispersion de particules de polymère ainsi préparée stable dans l'isododécane. Les caractéristiques de ce polymère et des particules formées par ledit polymère sont les suivantes :

Granulométrie : 46 mm réalisée par Malvern Autosizer Lo-C à 25°C ;
Extrait sec : 50% dans l'isododécane réalisé par thermobalance ;
Masse moléculaire Poids Mw : 119 200 ;
Masse moléculaire nombre Mn : 31 900 ;
Indice de polydispersité (Mw/Mn) : 3,74 ;
Masse moléculaire du macromonomère utilisé Mw : 4000 ;
Température de transition vitreuse < 40°C (mesurée par analyse thermo-mécanique dynamique (DMTA)).

*Préparation de la composition

La composition comprend les ingrédients suivants :

| Constituants | Quantité |
|---|---|
| Stéarate de glycéryle et Stéarate PEG-100 | 2 g |
| Tartrate de dimyristyle et alcool cétéarylique et $C_{12}$-$C_{15}$-pareth-7 et PPG-25-laureth-25 | 1,50 g |
| Cyclohexasiloxane | 5,39 g |
| Alcool stéarylique | 1 g |
| Eau | 66, 75 g |
| Phénoxyéthanol | 1 g |
| Séquestrant | 0,05 g |
| Polyacrylamide (Hostacerin AMPS de Clariant) | 0,40 g |
| Gomme de xanthane | 0,20 g |
| Cosmo S40 (dispersion aqueuse de silice colloïdale) | 17,10 g |
| Dispersion de polymère préparée ci-dessus | 4,61 g |

La composition de cet exemple est préparée de la même façon que celle de l'exemple comparatif ci-dessus, cette préparation comportant en outre l'incorporation du polymère préparé ci-dessus à 40-45°C dans l'émulsion après l'introduction de la dispersion aqueuse de silice colloïdale.

EXEMPLE 2

Cet exemple illustre la préparation et l'utilisation d'un polymère formant une dispersion de particules dans un solvant siliconé, ledit polymère étant obtenu par polymérisation de l'acrylate de méthyle et du macromonomère correspondant au polydiméthylsiloxane monométhacryloxypropyl dans le cyclopentadiméthylsiloxane.

*Préparation de la dispersion

Dans un réacteur de 500 mL, on charge 50 g d'heptane, 50 g de cyclopentadiméthylsiloxane, 7,5 g d'acrylate de méthyle et 2,5 g de macromonomère du type polydiméthylsiloxane monométhacryloxypropyl et 0,8 g de tertio butyl peroxy-2-éthylhexanoate (Trigonox 21S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 40 g d'acrylate de méthyle et 0,5 g de Trigonox 21S.

On laisse ensuite le chauffage pendant 4 heures à 90°C puis on distille l'heptane du milieu réactionnel.

A l'issue de cette opération de distillation, on obtient une dispersion de particules de polymère ainsi préparée stable dans le cyclopentadiméthylsiloxane. Les caractéristiques de ce polymère et des particules formées par ledit polymère sont les suivantes :

Granulométrie : 162 mm réalisée par Malvern Autosizer Lo-C à 25°C ;
Extrait sec : 51,4 % dans le cyclopentadiméthylsiloxane réalisé par thermobalance ;
Masse moléculaire Poids Mw : 104 400 ;
Masse moléculaire nombre Mn : 28 500 ;
Indice de polydispersité (Mw/Mn) : 3,67 ;
Masse moléculaire du macromonomère utilisé Mw : 5000 ;
Température de transition vitreuse < 40°C (mesurée par analyse thermo-mécanique dynamique (DMTA)).

*Préparation de la composition

La composition comprend les ingrédients suivants :

| Constituants | Quantité |
|---|---|
| Stéarate de glycéryle et Stéarate PEG-100 | 2 g |
| Tartrate de dimyristyle et alcool cétéarylique et $C_{12}$-$C_{15}$-pareth-7 et PPG-25-laureth-25 | 1,50 g |
| Cyclohexasiloxane | 5,13 g |
| Alcool stéarylique | 1 g |
| Eau | 66,75 g |
| Phénoxyéthanol | 1 g |
| Séquestrant | 0,05 g |
| Polyacrylamide (Hostacerin AMPS de Clariant) | 0,40 g |
| Gomme de xanthane | 0,20 g |
| Cosmo S40 (dispersion aqueuse de silice colloïdale) | 17,10 g |
| Dispersion de polymère préparée ci-dessus | 4,87 g |

La composition de cet exemple est préparée de la même façon que celle de l'exemple comparatif ci-dessus, cette préparation comportant en outre l'incorporation du polymère préparé ci-dessus à 40-45°C dans l'émulsion après l'introduction de la dispersion aqueuse de silice colloïdale.

EXEMPLE 3

Cet exemple illustre la préparation d'une dispersion de particules solides d'un polymère dans un solvant carboné, obtenue par polymérisation d'acrylate de méthyle, d'acide acrylique et du macromonomère méthacrylate de polyéthylène/polybutylène (Kraton L-1253). Cette dispersion est utilisée comme agent tenseur dans le cadre de cette invention.
Le mode opératoire de préparation de cette dispersion particulier est le suivant :
Dans un réacteur de 500 mL, on charge 50 g d'heptane, 50 g d'isododécane, 3,5 g d'acrylate de méthyle, 2,5 g d'acide acrylique et 4 g du macromonomère méthacrylate de polyéthylène/polybutylène (Kraton L-1253) et 0,8 g de tertio-butyl-peroxy-2-éthylhexanoate (Trigonox 21S).
On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 17,5 g d'acrylate de méthyle, 22,5 g d'acide acrylique et 0,5 g de Trigonox 21S.
On laisse ensuite le chauffage pendant 4 heures à 90°C puis on distille l'heptane du milieu réactionnel.
A l'issue de cette opération de distillation, on obtient une dispersion de particules de polymère ainsi préparée stable dans l'isododécane. Les caractéristiques de ce polymère et des particules formées par ledit polymère sont les suivantes :

Granulométrie : 63 mm réalisée par Malvern Autosizer Lo-C à 25°C ;

Extrait sec : 53,6 % dans l'isododécane réalisé par thermobalance ;

Masse moléculaire du macromonomère utilisé Mw : 4000.

EXEMPLE 4 : Mise en évidence de l'effet tenseur rémanent selon le premier protocole

Le protocole de quantification de la rémanence de l'effet tenseur tel que défini ci-dessus a été mis en oeuvre pour les trois compositions de l'exemple comparatif et des exemples 1 et 2.
Ce protocole vise à quantifier le potentiel renforçateur des polymères sous forme de dispersions de l'exemple 1 (dans l'isododécane) et de l'exemple 2 (dans le cyclopentadiméthylsiloxane) une fois introduits dans une composition anti rides.
Les résultats obtenus sont regroupés dans le tableau ci-dessous.

| | $F_{rupt}$ (N) | $W_{rupt}$ ($J/m^2$) |
|---|---|---|
| Exemple comparatif | 0.18±0.02 | 19±3 |
| Exemple 1 (silice CS40 7% + 2.5% de la dispersion) | 0.40±0.01 | 146±11 |
| Exemple 2(silice CS40 7% + 2.5% de la dispersion) | 0.41±0.01 | 131±5 |

Ces résultats mettent en évidence le rôle renforçateur des 2 dispersions étudiées en présence d'un agent tenseur. Ce rôle de renfort s'illustre par une augmentation de la force et de l'énergie à rupture.

EXEMPLE 5 : Mise en évidence de l'effet tenseur rémanent selon le deuxième protocole

Le second protocole a été mis en oeuvre pour les compositions suivantes :

- une composition constituée uniquement d'une dispersion d'un polymère de l'exemple 3 dans l'isododécane (intitulée composition a) ;
- une composition constituée d'un mélange dans un rapport 90/10 dans l'isododécane, d'une dispersion de l'exemple 3 et d'une dispersion de l'exemple 1 (intitulée composition b) ;
- une composition constituée uniquement d'un agent tenseur en dispersion dans le cyclopentadiméthylsiloxane, cet agent tenseur étant un copolymère de méthacrylate d'isobutyle, d'acide acrylique et d'un macromonomère siliconé CTFA (tel que défini ci-dessus) à 23% en poids (commercialisé sous la référence SA 70 par 3M) (intitulée composition c) ;
- une composition constituée d'un mélange, dans un rapport 90/10 dans le cyclopentadiméthylsiloxane d'un agent tenseur SA70 et d'une dispersion telle que préparée dans l'exemple 2 (composition d).

Les résultats obtenus selon ce second protocole sont regroupés dans le tableau suivant.

| | ET(%) | $ET_r$ (%) |
|---|---|---|
| Composition a | 42±5 | 0 |
| Composition b | 62±5 | 45±6 |
| Composition c | 80±5 | 20±4 |
| Composition d | 80±5 | 50±5 |

On peut constater que les compositions comportant une dispersion conforme à la présente invention présentent une grandeur $ET_r$ plus élevée que les compositions ne comportant pas une telle dispersion, ce qui signifie que les dispersions de l'invention induisent une rémanence de l'effet tenseur engendré par l'agent tenseur.

EXEMPLE 6 : Effet sur le blanchiment de la peau

Les compositions cosmétiques correspondant à l'exemple comparatif et l'exemple 2 ci-dessus ont été étalées à l'aide d'un tire-film mécanique sur une carte de contraste (Prufkarte type 24/5-250 $cm^2$) commercialisée par la société Erichsen (épaisseur du film : 30 μm) On a ensuite séché les compositions pendant 3 heures à une température de 20°C et des photographies des zones traitées ont été prises.

On a remarqué dans le cas de la composition de l'exemple comparatif l'apparition de dépôts blancs inesthétiques sur la zone traitée. Dans le cas de la composition de l'exemple 2 selon l'invention, de tels dépôts inesthétiques sont absents.

**REFERENCES CITEES**

[1] FR-A-2 758 083 ;
[2] US-6,139,322 ;

[3] US-6,465,001 ;
[4] US-5,349,003 ;
[5] EP-1 038 519 ;
[6] FR-2 819 429 ;
[7] FR-03 11346;
[8] Food Gels, Peter Harris, Elsevier 1989, Chap. 3 ;
[9] Food Gels, Peter Harris, Elsevier 1989, Chap. 1 ;
[10] Food Gels, Peter Harris, Elsevier 1989, Chap. 6 ;
[11] FR-2 829 025 ;
[12] Kirk-Othmer Encyclopedia Of Chemical Technology, 3ème édition, volume 21, p.492-507, Wiley Interscience, 1983 ;
[13] Light Scattering by Small Particles, Wiley, New York, 1957, Chap. 9 et 10;
[14] Polymer Handbook, 3éme édition, Chapitre VII, p.519-559 ;
[15] J.Paint Technol. 39, 105 (1967) ;
[16] EP 895467 ;
[17] EP 96459 ;
[18] Polymer Letters, Vol 5, page 477-481 (1967) ;
[19] US 5,625,005.

## Revendications

**1.** Procédé cosmétique pour atténuer les rides d'une peau ridée comprenant une étape consistant à appliquer sur ladite peau ridée une composition cosmétique comprenant, dans un milieu physiologiquement acceptable adapté à une application topique sur la peau du visage :

- de 0,1 à 20% en poids d'au moins un agent tenseur, par rapport au poids total de la composition ; et
- au moins une dispersion dans une phase grasse liquide de particules solides d'un polymère éthylénique greffé.

**2.** Procédé cosmétique selon la revendication 1, dans lequel l'agent tenseur est présent à une teneur allant de 1 à 10% du poids total de la composition.

**3.** Procédé cosmétique selon la revendication 1 ou 2, dans lequel la dispersion est présente dans la composition à une teneur allant de 0,01 à 20%, de préférence de 1 à 10% du poids total de la composition.

**4.** Procédé cosmétique selon l'une quelconque des revendications 1 à 3, dans lequel la phase grasse liquide est présente dans la composition à une teneur allant de 0,5 à 80% du poids total de la composition, de préférence de 1 à 55%, de préférence encore de 1 à 25%.

**5.** Procédé cosmétique selon l'une quelconque des revendications 1 à 4, dans lequel l'agent tenseur est un agent produisant à une concentration de 7% dans l'eau une rétraction du *stratum corneum* isolé, mesuré avec un extensomètre, de plus de 1% et de préférence supérieure à 1,5% à 30°C et sous une humidité relative de 40%.

**6.** Procédé cosmétique selon l'une quelconque des revendications 1 à 5, dans lequel l'agent tenseur est choisi parmi les polymères synthétiques, les polymères d'origine naturelle, les silicates mixtes, les microparticules de cire, les particules colloïdales de charges inorganiques et les mélanges de ceux-ci.

**7.** Procédé cosmétique selon la revendication 6, dans lequel les polymères synthétiques sont choisis parmi :

- les polymères et copolymères de polyuréthanne ;
- les polymères et copolymères acryliques ;
- les polymères d'acide isophtalique sulfoné ;
- les polymères siliconés greffés ;
- les polymères hydrosolubles ou hydrodispersibles comprenant des unités hydrosolubles ou hydrodispersibles et des unités à LCST ;
- les polymères éthyléniques séquencés linéaires filmogènes non élastomères et non hydrosolubles présentant un module dynamique de conservation E' à 1Hz et à 22°C supérieur à 200 MPa ;
- les polymères éthyléniques greffés en dispersion de particules solides dans une phase grasse liquide présentant une température de transition vitreuse supérieure à 40°C ;
- et les mélanges de ceux-ci.

8. Procédé cosmétique selon la revendication 6, dans lequel les polymères d'origine naturelle sont choisis parmi les protéines végétales et hydrolysats de protéines végétales, les polysaccharides d'origine végétale sous forme de microgels, les latex d'origine végétale et les mélanges de ceux-ci.

9. Procédé cosmétique selon l'une quelconque des revendications 1 à 8, dans lequel ledit polymère éthylénique greffé comprend un squelette insoluble dans ladite phase grasse liquide, et une partie soluble dans ladite phase grasse liquide constituée de chaînes latérales liées de manière covalente audit squelette.

10. Procédé cosmétique selon l'une quelconque des revendications 1 à 9, dans lequel le polymère éthylénique greffé est un polymère acrylique greffé.

11. Procédé cosmétique selon la revendication 10, dans lequel le polymère acrylique greffé est susceptible d'être obtenu par polymérisation radicalaire dans ladite phase grasse liquide :

- d'au moins un monomère acrylique, et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 et la teneur en macromonomère polymérisé représentant de 0,05 à 20 % en poids du polymère.

12. Procédé cosmétique selon la revendication 11, dans lequel le ou les monomères acryliques sont choisis parmi les monomères dont l'homopolymère est insoluble dans la phase grasse liquide considérée, c'est-à-dire que l'homopolymère est sous forme solide à une concentration supérieure ou égale à 5% en poids à température ambiante (20°C) dans ladite phase grasse liquide.

13. Procédé cosmétique selon la revendication 11 ou 12, dans lequel le ou les monomères acryliques sont choisis parmi les monomères suivants :

- les (méth)acrylates de formule (IV) suivante :

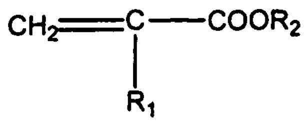

(IV)

dans laquelle :

- $R_1$ désigne un atome d'hydrogène ou un groupe méthyle ;
- $R_2$ représente un groupe choisi parmi :

  - un groupe alkyle, linéaire ou ramifié, comprenant de 1 à 6 atomes de carbone, ledit groupe pouvant comporter dans sa chaîne un ou plusieurs hétéroatomes choisis parmi O, N et S ; et/ou pouvant comporter un ou plusieurs substituants choisis parmi - OH, les atomes d'halogène (F, Cl, Br, I) et -NR' R" avec R' et R", identiques ou différents, étant choisis parmi les groupes alkyles, linéaires ou ramifiées, comportant de 1 à 4 atomes de carbone ; et/ou pouvant être substitué par au moins un groupe polyoxyalkylène, en particulier un groupe alkylène comportant de 1 à 4 atomes de carbone, notamment un groupe polyoxyéthylène et/ou polyoxypropylène, ledit groupe polyoxyalkylène étant constitué par la répétition de 5 à 30 motifs oxyalkylène ;
  - un groupe alkyle cyclique comprenant de 3 à 6 atomes de carbone, ledit groupe pouvant comporter dans sa chaîne un ou plusieurs hétéroatomes choisis parmi O, N et S, et pouvant comporter un ou plusieurs substituants choisis dans un groupe constitué par OH, les atomes d'halogène (F, Cl, Br, I) ;

- les (méth)acrylamides de formule (V) suivante :

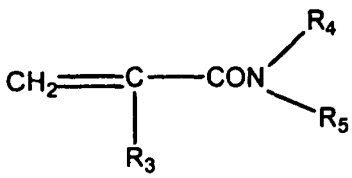

(V)

dans laquelle :

- $R_3$ désigne un atome d'hydrogène ou un groupe méthyle ;
- $R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R" avec R' et R", identiques ou différents, étant choisis parmi les groupes alkyles, linéaires ou ramifiées, comportant de 1 à 4 atomes de carbone; ou
- $R_4$ représente un atome d'hydrogène et $R_5$ représente un groupe 1,1-diméthyl-3-oxobutyle ;

- les monomères (méth)acryliques comprenant au moins une fonction acide carboxylique, phosphorique ou sulfonique ;

lesdits monomères pouvant être sous forme de sels.

**14.** Procédé cosmétique selon l'une quelconque des revendications 11 à 13, dans lequel le ou les monomères additionnels vinyliques non acryliques sont choisis dans le groupe constitué par :

- les esters de vinyle de formule (VI) suivante:

$$R_6\text{-COO-CH=CH}_2 \quad \text{(VI)}$$

dans laquelle :

- $R_6$ représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;
- les monomères vinyliques non acryliques comprenant au moins une fonction acide carboxylique ou sulfonique, tel que l'acide crotonique, l'anhydride maléique, l'acide itaconique, l'acide fumarique, l'acide maléique, l'acide styrènesulfonique, l'acide vinylbenzoïque, l'acide vinylphosphorique et les sels de ceux-ci ;
- les monomères vinyliques non acryliques comprenant au moins une fonction amine tertiaire, tel que la 2-vinylpyridine, la 4-vinylpyridine ;
- et les mélanges de ceux-ci.

**15.** Procédé cosmétique selon l'une quelconque des revendications 11 à 14, dans lequel le ou les monomères acryliques représentent de 50 à 100 % en poids, de préférence de 55 à 100 % en poids, préférentiellement de 60 à 100 % en poids du mélange constitué du ou des monomères acryliques et du ou des monomères vinyliques non acryliques éventuels.

**16.** Procédé cosmétique selon l'une quelconque des revendications 11 à 13, dans lequel le polymère acrylique greffé ne contient pas de monomères additionnels vinyliques non acryliques.

**17.** Procédé cosmétique selon l'une quelconque des revendications 11 à 16, dans lequel le macromonomère comporte à une de ses extrémités un groupe terminal polymérisable choisi parmi un groupe vinyle, un groupe (méth)acrylate.

**18.** Procédé cosmétique selon l'une quelconque des revendications 11 à 17, dans lequel le macromonomère présente

une masse moléculaire moyenne en poids (Mw) allant de 200 à 100 000, de préférence allant de 500 à 50 000, préférentiellement allant de 800 à 20 000, plus préférentiellement allant de 800 à 10 000 et encore plus préférentiellement allant de 800 à 6000.

**19.** Procédé cosmétique selon l'une quelconque des revendications 11 à 18, dans lequel le macromonomère polymérisé représente de 0,1 à 15% en poids du poids total du polymère, de préférence de 0,2 à 10% en poids, et de préférence encore de 0,3 à 8% en poids.

**20.** Procédé cosmétique selon l'une quelconque des revendications 11 à 19, dans lequel le macromonomère est choisi parmi les macromonomères dont l'homopolymère est soluble dans la phase grasse liquide considérée, c'est-à-dire complètement dissous à une concentration supérieure ou égale à 5% en poids et à température ambiante (20°C) dans ladite phase grasse liquide.

**21.** Procédé cosmétique selon l'une quelconque des revendications 1 à 20, dans lequel ladite phase grasse liquide comprend au moins un composé liquide non aqueux choisi dans le groupe constitué par :

- les composés organiques liquides ayant un paramètre de solubilité global selon l'espace de solubilité de Hansen inférieur ou égal à 18 $(MPa)^{1/2}$, de préférence inférieur ou égal à 17 $(MPa)^{1/2}$ ;
- les monoalcools ayant un paramètre de solubilité global selon l'espace de solubilité de Hansen inférieur ou égal à 20 $(MPa)^{1/2}$ ; et
- les mélanges de ceux-ci.

**22.** Procédé cosmétique selon la revendication 21, dans lequel la phase grasse liquide est une phase grasse non siliconée, ladite phase grasse non siliconée ne contenant pas de composés organiques liquides siliconés ou comprenant moins de 50% en poids de composés organiques liquides siliconés.

**23.** Procédé cosmétique selon la revendication 22, dans lequel la phase grasse non siliconée comprend au moins 50% en poids d'au moins un composé liquide organique non siliconé choisi parmi :

- les composés liquides organiques non siliconés ayant un paramètre de solubilité global selon l'espace de solubilité de Hansen inférieur ou égal à 18 $(MPa)^{1/2}$, de préférence inférieur ou égal à 17 $(MPa)^{1/2}$ ;
- les monoalcools liquides ayant un paramètre de solubilité global selon l'espace de solubilité de Hansen inférieur ou égal à 20 $(MPa)^{1/2}$ ; et
- leurs mélanges.

**24.** Procédé cosmétique selon la revendication 23, dans lequel le composé liquide non siliconé ayant un paramètre de solubilité global selon l'espace de solubilité de Hansen inférieur ou égal à 18 $(MPa)^{1/2}$ , de préférence inférieur ou égal à 17 $(MPa)^{1/2}$ est choisi parmi :

- les huiles naturelles ou synthétiques, carbonées, hydrocarbonées, fluorées, éventuellement ramifiées, seules ou en mélange ;
- les alcanes linéaires, ramifiées et/ou cycliques, éventuellement volatils ;
- les esters et notamment les esters linéaires, ramifiés ou cycliques, ayant au moins 6 atomes de carbone, notamment ayant de 6 à 30 atomes de carbone ;
- les éthers et notamment les éthers ayant au moins 6 atomes de carbone, notamment ayant de 6 à 30 atomes de carbone ;
- les cétones et notamment les cétones ayant au moins 6 atomes de carbone, notamment ayant de 6 à 30 atomes de carbone.

**25.** Procédé cosmétique selon la revendication 23, dans lequel les monoalcools ayant un paramètre de solubilité global selon l'espace de solubilité de Hansen inférieur ou égal à 20 $(MPa)^{1/2}$ sont choisis dans le groupe formé par les monoalcools liquides gras aliphatiques, saturés ou insaturés, ayant au moins 6 atomes de carbone.

**26.** Procédé cosmétique selon l'une quelconque des revendications 23 à 25, dans lequel le ou les macromocomères sont des macromonomères carbonés.

**27.** Procédé cosmétique selon la revendication 26, dans lequel le macromonomère carboné est choisi parmi :

- (i) les homopolymères et les copolymères (méth)acrylate d'alkyle linéaire ou ramifié en C8-C22, présentant un groupe terminal polymérisable choisi parmi les groupes vinyle ou (méth)acrylate ;
- (ii) les polyoléfines ayant un groupe terminal à insaturation éthylénique, en particulier ayant un groupement terminal (méth)acrylate.

**28.** Procédé cosmétique selon la revendication 26, dans lequel le macromonomère carboné est choisi parmi :

- (i) les macromonomères de poly(acrylate d'éthyl-2 hexyle) à extrémité mono(méth)acrylate ; les macromonomères de poly(acrylate de dodécyle) à extrémité mono(méth)acrylate ; les macromonomères de poly(méthacrylate de dodécyle) ; les macromonomères de poly(acrylate de stéaryle) à extrémité mono(méth)acrylate ; les macromonomères de poly (méthacrylate de stéaryle) à extrémité mono(méth)acrylate ;
- (ii) les macromonomères de polyéthylène, les macromonomères de polypropylène, les macromonomères de copolymère polyéthylène/polypropylène, les macromonomères de copolymère polyéthylène/polybutylène, les macromonomères de polyisobutylène, les macromonomères de polybutadiène, les macromonomères de polyisoprène, les macromonomères de polybutadiène, les macromonomères de poly(éthylène/butylène)-polyisoprène, ces macromonomères ayant un groupement terminal (méth)acrylate.

**29.** Procédé cosmétique selon la revendication 27 ou 28, dans lequel le macromonomère carboné est choisi parmi :

- (i) les macromonomères de poly(acrylate d'éthyl-2-hexyle) à extrémité mono(méth)acrylate, les macromonomères de poly(acrylate de dodécyle) à extrémité mono(méth)acrylate ;
- (ii) le méthacrylate de poly(éthylène/butylène).

**30.** Procédé cosmétique selon l'une quelconque des revendications 1 à 29, dans lequel la dispersion est une dispersion obtenue par polymérisation de l'acrylate de méthyle et du macromonomère méthacrylate de polyéthylène/polybutylène dans l'isododécane et l'agent tenseur est une dispersion colloïdale de silice.

**31.** Procédé cosmétique selon l'une quelconque des revendications 1 à 29, dans lequel la dispersion est une dispersion obtenue par polymérisation de l'acrylate de méthyle et du macromonomère méthacrylate de polyéthylène/polybutylène dans l'isododécane et l'agent tenseur est une dispersion par polymérisation dans l'isododécane d'acrylate de méthyle, d'acide acrylique et du macromonomère méthacrylate de polyéthylène/polybutylène.

**32.** Procédé cosmétique selon la revendication 21, dans lequel la phase grasse liquide est une phase grasse liquide siliconée.

**33.** Procédé cosmétique selon la revendication 32, dans lequel la phase grasse liquide siliconée comprend au moins 50 % en poids d'au moins un composé liquide organique siliconé choisi parmi les composés liquides organiques siliconés ayant un paramètre de solubilité global selon l'espace de solubilité de Hansen inférieur ou égal à 17 $(MPa)^{1/2}$.

**34.** Procédé cosmétique selon la revendication 32 ou 33, dans lequel la phase grasse liquide comprend une huile siliconée volatile.

**35.** Procédé cosmétique selon la revendication 34, dans lequel l'huile siliconée volatile est choisie parmi l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane, et leurs mélanges.

**36.** Procédé cosmétique selon la revendication 32 ou 33, dans lequel la phase grasse liquide comprend une huile siliconée non volatile.

**37.** Procédé cosmétique selon la revendication 36, dans lequel l'huile siliconée non volatile est choisie parmi les polydialkylsiloxanes non volatils; les polydiméthylsiloxanes comportant des groupements alkyle, alcoxy ou phényle, pendant ou en bout de chaîne siliconée, groupements ayant de 2 à 24 atomes de carbone; les silicones phénylées ; les polysiloxanes modifiés par des acides gras en $C_8$-$C_{20}$, des alcools gras en $C_8$-$C_{20}$ ou des polyoxyalkylènes (notamment polyoxyéthyléne et/ou polyoxypropylène); les polysiloxanes aminées ; les polysiloxanes à groupements hydroxyles; les polysiloxanes fluorés comportant un groupement fluoré pendant ou en bout de chaîne siliconée ayant de 1 à 12 atomes de carbone dont tout ou partie des hydrogène sont substitués par des atomes de fluor ; et leurs mélanges.

**38.** Procédé cosmétique selon l'une quelconque des revendications 32 à 37, dans lequel la phase grasse liquide comprend moins de 50 % en poids de composés organiques liquides non siliconés.

**39.** Procédé cosmétique selon l'une quelconque des revendications 32 à 37, dans lequel la phase grasse liquide ne contient pas de composés organiques liquides non siliconés.

**40.** Procédé cosmétique selon l'une quelconque des revendications 32 à 39, dans lequel le macromonomère est un macromonomère siliconé.

**41.** Procédé cosmétique selon la revendication 40, dans lequel le macromonomère siliconé est un macromonomère organopolysiloxane, et de préférence un macromonomère polydiméthylsiloxane.

**42.** Procédé cosmétique selon l'une quelconque des revendications 32 à 41, dans lequel le polymère acrylique greffé est susceptible d'être obtenu par polymérisation radicalaire dans ladite phase grasse liquide :

- d'un monomère acrylique principal choisi parmi les (méth)acrylates d'alkyle en $C_1$-$C_3$, seuls ou en mélange, et éventuellement d'un ou plusieurs monomères acryliques additionnels choisis parmi l'acide acrylique, l'acide méthacrylique et les (méth)acrylates d'alkyle de formule (VII) :

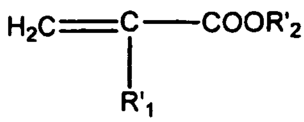

(VII)

dans lequel :

- R'$_1$ désigne un atome d'hydrogène ou un groupe méthyle ;
- R'$_2$ représente
  - un groupe alkyle linéaire ou ramifié comprenant de 1 à 6 atomes de carbone, ledit groupe comportant dans sa chaîne un ou plusieurs atomes d'oxygène et/ou comportant un ou plusieurs substituants choisis parmi
  - OH, les atomes d'halogène (F, Cl, Br, I) et -NR'R" avec R' et R", identiques ou différents, choisis parmi les alkyles linéaires ou ramifiés en $C_1$-$C_3$ :
  - un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I) ;

et leurs sels, pour former ledit squelette insoluble ;

- et d'un macromonomère siliconé.

**43.** Procédé cosmétique selon la revendication 42, dans lequel R'$_2$ désigne un groupe choisi parmi les groupes méthoxyéthyle, éthoxyéthyle, trifluoroéthyle; 2-hydroxyéthyle, 2-hydroxypropyle, diméthylaminoéthyle, diéthylaminoéthyle, diméthylaminopropyle.

**44.** Procédé cosmétique selon la revendication 42, dans lequel le monomère acrylique principal est choisi parmi le (méth)acrylate de méthyle, le (méth)acrylate d'éthyle, le (méth)acrylate de n-propyle, le (méth)acrylate d'isopropyle, et leurs mélanges.

**45.** Procédé cosmétique selon la revendication 44, dans lequel le monomère acrylique principal est choisi parmi l'acrylate de méthyle, le méthacrylate de méthyle, le méthacrylate d'éthyle.

**46.** Procédé cosmétique selon la revendication 42, dans lequel le monomère acrylique additionnel est choisi parmi l'acide (méth)acrylique, le (méth)acrylate de méthoxyéthyle, le (méth)acrylate d'éthoxyéthyle, le méthacrylate de trifluoroéthyle, le méthacrylate de diméthylaminoéthyle, le méthacrylate de diéthylaminoéthyle, le (méth)acrylate de 2-hydroxypropyle, le (méth)acrylate de 2-hydroxyéthyle, leurs sels et leurs mélanges.

**47.** Procédé cosmétique selon la revendication 46, dans lequel le monomère acrylique additionnel est choisi parmi l'acide acrylique, l'acide méthacrylique.

**48.** Procédé cosmétique selon l'une quelconque des revendications 42 à 47, dans lequel le macromonomère siliconé est choisi parmi les polydiméthylsiloxanes à groupement terminal mono (méth)acrylate.

**49.** Procédé cosmétique selon la revendication 48, dans lequel le macromonomère siliconé répond à la formule (VIII) suivante :

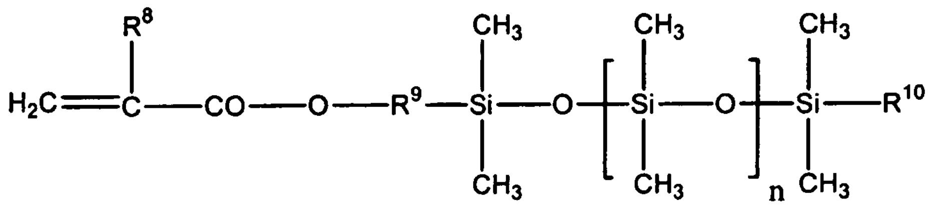

(VIII)

dans laquelle :

- $R^8$ représente un atome d'hydrogène ou un groupe méthyle ;
- $R^9$ représente un groupe hydrocarboné divalent ayant de 1 à 10 atomes de carbone et comprend éventuellement une ou deux liaisons éther -O- ;
- $R^{10}$ représente un groupe alkyle ayant de 1 à 10 atomes de carbone, notamment de 2 à 8 atomes de carbone ;
- n représente un nombre entier allant de 1 à 300, de préférence allant de 3 à 200, et préférentiellement allant de 5 à 100.

**50.** Procédé cosmétique selon l'une quelconque des revendications 33 à 49, dans lequel la dispersion est obtenue par polymérisation de l'acrylate de méthyle et du macromonomère monométhacryloxypropylpolydiméthylsiloxane dans le cyclopentadiméthylsiloxane et l'agent tenseur est une dispersion colloïdale de silice.

**51.** Procédé cosmétique selon l'une quelconque des revendications 1 à 50, dans lequel le polymère greffé a une masse moléculaire moyenne en poids (Mw) comprise entre 10 000 et 300 000, notamment entre 20 000 et 200 000, mieux encore entre 25 000 et 150 000.

**52.** Procédé cosmétique selon l'une quelconque des revendications 1 à 51, dans lequel les particules de polymère greffé ont une taille moyenne allant de 10 à 400 nm, de préférence allant de 20 à 200 nm.

**53.** Procédé cosmétique selon l'une quelconque des revendications 1 à 52, dans lequel la composition est appliquée sur le contour de l'oeil.

**54.** Procédé cosmétique selon l'une quelconque des revendications 1 à 53, dans lequel la composition est une composition de soin ou une composition de maquillage.

**55.** Composition cosmétique comprenant, dans un milieu physiologiquement acceptable adapté à une application topique sur la peau du visage :

- de 0,1 à 20% en poids d'au moins un agent tenseur, par rapport au poids total de la composition, ledit agent tenseur étant sous forme de particules colloïdales de charges inorganiques ; et

- au moins une dispersion dans une phase grasse liquide de particules solides d'un polymère éthylénique greffé.

**56.** Utilisation d'une dispersion de particules solides d'un polymère éthylénique greffé tel que défini selon l'une quelconque des revendications 1 à 52 pour améliorer la rémanence de l'effet tenseur procuré par un agent tenseur tel que défini selon l'une quelconque des revendications 1 à 7.

**57.** Utilisation d'une dispersion de particules solides d'un polymère éthylénique greffé tel que défini selon l'une quelconque des revendications 1 à 52 dans une composition cosmétique comprenant en tant qu'agent tenseur une dispersion aqueuse de particules colloïdales inorganiques, en particulier de silice, pour prévenir le blanchiment de la peau.

## Claims

**1.** Cosmetic process for softening the wrinkles of wrinkled skin comprising a stage consisting in applying, to said wrinkled skin, a cosmetic composition, comprising, in a physiologically acceptable medium suitable for topical application to the skin of the face:

- from 0.1 to 20% by weight of at least one tensioning agent, with respect to the total weight of the composition, and
- at least one dispersion in a liquid fatty phase of solid particles of a grafted ethylenic polymer.

**2.** Cosmetic process according to Claim 1, in which the tensioning agent is present at a content ranging from 1 to 10% of the total weight of the composition.

**3.** Cosmetic process according to Claim 1 or 2, in which the dispersion is present in the composition at a content ranging from 0.01 to 20%, preferably from 1 to 10%, of the total weight of the composition.

**4.** Cosmetic process according to any one of Claims 1 to 3, in which the liquid fatty phase is present in the composition at a content ranging from 0.5 to 80% of the total weight of the composition, preferably from 1 to 55%, more preferably from 1 to 25%.

**5.** Cosmetic process according to any one of Claims 1 to 4, in which the tensioning agent is an agent producing, at a concentration of 7% in water, a retraction of the isolated *stratum corneum,* measured with an extensometer, of more than 1% and preferably of more than 1.5% at 30°C under a relative humidity of 40%.

**6.** Cosmetic process according to any one of Claims 1 to 5, in which the tensioning agent is chosen from synthetic polymers, polymers of natural origin, mixed silicates, wax microparticles, colloidal particles of inorganic fillers and the mixtures of these.

**7.** Cosmetic process according to Claim 6, in which the synthetic polymers are chosen from:

- polyurethane polymers and copolymers;
- acrylic polymers and copolymers;
- polymers of sulphoisophthalic acid;
- grafted silicone polymers;
- water-soluble or water-dispersible polymers comprising water-soluble or water-dispersible units and LCST units;
- non-elastomeric and water-insoluble film-forming linear ethylenic block polymers exhibiting a dynamic storage modulus E' at 1 Hz and at 22°C of greater than 200 MPa;
- grafted ethylenic polymers as a dispersion of solid particles in a liquid fatty phase exhibiting a glass transition temperature of greater than 40°C;
- and mixtures of these.

**8.** Cosmetic process according to Claim 6, in which the polymers of natural origin are chosen from plant proteins and plant protein hydrolysates, polysaccharides of plant origin in the form of microgels, latexes of plant origin and mixtures of these.

**9.** Cosmetic process according to any one of Claims 1 to 8, in which the said grafted ethylenic polymer comprises a

backbone which is insoluble in the said liquid fatty phase and a part which is soluble in the said liquid fatty phase composed of side chains covalently bonded to the said backbone.

**10.** Cosmetic process according to any one of Claims 1 to 9, in which the grafted ethylenic polymer is a grafted acrylic polymer.

**11.** Cosmetic process according to Claim 10, in which the grafted acrylic polymer is capable of being obtained by radical polymerization in the said liquid fatty phase:

- of at least one acrylic monomer and optionally of at least one additional non-acrylic vinyl monomer, in order to form the said insoluble backbone; and
- of at least one macromonomer comprising a polymerizable end group, in order to form the side chains, the said macromonomer having a weight-average molecular weight of greater than or equal to 200 and the content of polymerized macromonomer representing from 0.05 to 20% by weight of the polymer.

**12.** Cosmetic process according to Claim 11, in which the acrylic monomer or monomers are chosen from monomers, the homopolymer of which is insoluble in the liquid fatty phase under consideration, that is to say that the homopolymer is in the solid form at a concentration of greater than or equal to 5% by weight at ambient temperature (20°C) in the said liquid fatty phase.

**13.** Cosmetic process according to Claim 11 or 12, in which the acrylic monomer or monomers are chosen from the following monomers:

- (meth)acrylates of following formula (IV):

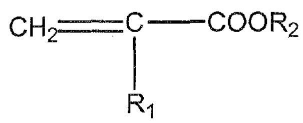

(IV)

in which:

- $R_1$ denotes a hydrogen atom or a methyl group;
- $R_2$ represents a group chosen from:
    - a linear or branched alkyl group comprising from 1 to 6 carbon atoms, it being possible for the said group to comprise, in its chain, one or more heteroatoms chosen from 0, N and S; and/or it being possible for the said group to comprise one or more substituents chosen from
    - OH, halogen atoms (F, Cl, Br, I) and -NR'R" with R' and R", which are identical or different, being chosen from linear or branched alkyl groups comprising from 1 to 4 carbon atoms; and/or it being possible for the said group to be substituted by at least one polyoxyalkylene group, in particular one alkylene group comprising from 1 to 4 carbon atoms, especially one polyoxyethylene and/or polyoxypropylene group, the said polyoxyalkylene group being composed of the repetition of 5 to 30 oxyalkylene units;
    - a cyclic alkyl group comprising from 3 to 6 carbon atoms, it being possible for the said group to comprise, in its chain, one or more heteroatoms chosen from O, N and S and it being possible for the said group to comprise one or more substituents chosen from the group consisting of OH and halogen atoms (F, Cl, Br, I);

- (meth)acrylamides of following formula (V):

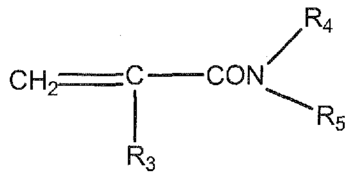

(V)

in which:

- $R_3$ denotes a hydrogen atom or a methyl group;
- $R_4$ and $R_5$, which are identical or different, represent a hydrogen atom or a linear or branched alkyl group comprising from 1 to 6 carbon atoms which can comprise one or more substituents chosen from -OH, halogen atoms (F, Cl, Br, I) and -NR'R" with R' and R", which are identical or different, being chosen from linear or branched alkyl groups comprising from 1 to 4 carbon atoms; or
- $R_4$ represents a hydrogen atom and $R_5$ represents a 1,1-dimethyl-3-oxobutyl group;

- (meth)acrylic monomers comprising at least one carboxylic, phosphoric or sulphonic acid functional group;

it being possible for the said monomers to be in the form of salts.

**14.** Cosmetic process according to any one of Claims 11 to 13, in which the additional non-acrylic vinyl monomer or monomers are chosen from the group consisting of:

- vinyl esters of following formula (VI):

$$R_6\text{-COO-CH=CH}_2 \qquad (VI)$$

in which:

- $R_6$ represents a linear or branched alkyl group comprising from 1 to 6 carbon atoms or a cyclic alkyl group comprising from 3 to 6 carbon atoms and/or an aromatic group, for example of benzene, anthracene and naphthalene type;
- non-acrylic vinyl monomers comprising at least one carboxylic or sulphonic acid functional group, such as crotonic acid, maleic anhydride, itaconic acid, fumaric acid, maleic acid, styrenesulphonic acid, vinylbenzoic acid, vinylphosphoric acid and the salts of these;
- non-acrylic vinyl monomers comprising at least one tertiary amine functional group, such as 2-vinylpyridine or 4-vinylpyridine;
- and mixtures of these.

**15.** Cosmetic process according to any one of Claims 11 to 14, in which the acrylic monomer or monomers represent from 50 to 100% by weight, preferably from 55 to 100% by weight, preferentially from 60 to 100% by weight, of the mixture composed of the acrylic monomer or monomers and of the optional non-acrylic vinyl monomer or monomers.

**16.** Cosmetic process according to any one of Claims 11 to 13, in which the grafted acrylic polymer does not comprise additional non-acrylic vinyl monomers.

**17.** Cosmetic process according to any one of Claims 11 to 16, in which the macromonomer comprises, at one of its ends, a polymerizable end group chosen from a vinyl group or a (meth) acrylate group.

**18.** Cosmetic process according to any one of Claims 11 to 17, in which the macromonomer exhibits a weight-average molecular weight (Mw) ranging from 200 to 100 000, preferably ranging from 500 to 50 000, preferentially ranging from 800 to 20 000, more preferentially ranging from 800 to 10 000 and more preferentially still ranging from 800 to 6000.

**19.** Cosmetic process according to any one of Claims 11 to 18, in which the polymerized macromonomer represents

from 0.1 to 15% by weight of the total weight of the polymer, preferably from 0.2 to 10% by weight and more preferably from 0.3 to 8% by weight.

**20.** Cosmetic process according to any one of Claims 11 to 19, in which the macromonomer is chosen from macromonomers, the homopolymer of which is soluble in the liquid fatty phase under consideration, that is to say completely dissolved at a concentration of greater than or equal to 5% by weight and at ambient temperature (20°C) in the said liquid fatty phase.

**21.** Cosmetic process according to any one of Claims 1 to 20, in which the said liquid fatty phase comprises at least one non-aqueous liquid compound chosen from the group consisting of:

- liquid organic compounds having an overall solubility parameter according to the Hansen solubility space of less than or equal to 18 $(MPa)^{½}$, preferably of less than or equal to 17 $(MPa)^{½}$;
- monoalcohols having an overall solubility parameter according to the Hansen solubility space of less than or equal to 20 $(MPa)^{½}$ ; and
- mixtures of these.

**22.** Cosmetic process according to Claim 21, in which the liquid fatty phase is a non-silicone fatty phase, said non-silicone fatty phase not comprising silicone liquid organic compounds or comprising less than 50% by weight of silicone liquid organic compounds.

**23.** Cosmetic process according to Claim 22, in which the non-silicone fatty phase comprises at least 50% by weight of at least one non-silicone liquid organic compound chosen from:

- non-silicone liquid organic compounds having an overall solubility parameter according to the Hansen solubility space of less than or equal to 18 $(MPa)^{1/2}$, preferably of less than or equal to 17 $(MPa)^{½}$;
- liquid monoalcohols having an overall solubility parameter according to the Hansen solubility space of less than or equal to 20 $(MPa)^{½}$; and
- their mixtures.

**24.** Cosmetic process according to Claim 23, in which the non-silicone liquid compound having an overall solubility parameter according to the Hansen solubility space of less than or equal to 18 $(MPa)^{½}$ preferably of less than or equal to 17 $(MPa)^{½}$, is chosen from:

- optionally branched, carbon, hydrocarbon or fluorinated, natural or synthetic oils, alone or as a mixture;
- optionally volatile, linear, branched and/or cyclic alkanes;
- esters and in particular linear, branched or cyclic esters having at least 6 carbon atoms, in particular having from 6 to 30 carbon atoms;
- ethers and in particular ethers having at least 6 carbon atoms, in particular having from 6 to 30 carbon atoms;
- ketones and in particular ketones having at least 6 carbon atoms, in particular having from 6 to 30 carbon atoms.

**25.** Cosmetic process according to Claim 23, in which the monoalcohols having an overall solubility parameter according to the Hansen solubility space of less than or equal to 20 $(MPa)^{½}$ are chosen from the group formed by saturated or unsaturated liquid aliphatic fatty monoalcohols having at least 6 carbon atoms.

**26.** Cosmetic process according to any one of Claims 23 to 25, in which the macromonomer or macromonomers are carbon macromonomers.

**27.** Cosmetic process according to Claim 26, in which the carbon macromonomer is chosen from:

- (i) linear or branched $C_8$-$C_{22}$ alkyl (meth)acrylate homopolymers and copolymers exhibiting a polymerizable end group chosen from vinyl or (meth)acrylate groups;
- (ii) polyolefins having an end group comprising ethylenic unsaturation, in particular having a (meth)acrylate end group.

**28.** Cosmetic process according to Claim 26, in which the carbon macromonomer is chosen from:

- (i) poly(2-ethylhexyl acrylate) macromonomers comprising a mono(meth)acrylate end; poly(dodecyl acrylate)

macromonomers comprising a mono(meth)acrylate end; poly(dodecyl methacrylate) macromonomers comprising a mono(meth)acrylate end; poly(stearyl acrylate) macromonomers comprising a mono(meth)acrylate end; poly(stearyl methacrylate) macromonomers comprising a mono(meth)acrylate end;
- (ii) polyethylene macromonomers, polypropylene macromonomers, polyethylene/polypropylene copolymer macromonomers, polyethylene/polybutylene copolymer macromonomers, polyisobutylene macromonomers, polybutadiene macromonomers, polyisoprene macromonomers, polybutadiene macromonomers, poly(ethylene/butylene)-polyisoprene macromonomers, these macromonomers having a (meth)acrylate end group.

**29.** Cosmetic process according to Claim 27 or 28, in which the carbon macromonomer is chosen from:

- (i) poly(2-ethylhexyl acrylate) macromonomers comprising a mono(meth)acrylate end, poly-(dodecyl acrylate) macromonomers comprising a mono(meth)acrylate end;
- (ii) poly(ethylene/butylene) methacrylate.

**30.** Cosmetic process according to any one of Claims 1 to 29, in which the dispersion is a dispersion obtained by polymerization of methyl acrylate and of the macromonomer polyethylene/ polybutylene methacrylate in isododecane and the tensioning agent is a colloidal silica dispersion.

**31.** Cosmetic process according to any one of Claims 1 to 29, in which the dispersion is a dispersion obtained by polymerization of methyl acrylate and of the macromonomer polyethylene/ polybutylene methacrylate in isododecane and the tensioning agent is a dispersion obtained by polymerization in isododecane of methyl acrylate, of acrylic acid and of the macromonomer polyethylene/polybutylene methacrylate.

**32.** Cosmetic process according to Claim 21, in which the liquid fatty phase is a silicone liquid fatty phase.

**33.** Cosmetic process according to Claim 32, in which the silicone liquid fatty phase comprises at least 50% by weight of at least one silicone liquid organic compound chosen from silicone liquid organic compounds having an overall solubility parameter according to the Hansen solubility space of less than or equal to 17 $(MPa)^{1/2}$.

**34.** Cosmetic process according to Claim 32 or 33, in which the liquid fatty phase comprises a volatile silicone oil.

**35.** Cosmetic process according to Claim 34, in which the volatile silicone oil is chosen from octamethylcyclotetrasiloxane, decamethylcyclopentasiloxane, dodecamethylcyclohexasiloxane, heptamethylhexyltrisiloxane, heptamethyloctyltrisiloxane, octamethyltrisiloxane, decamethyltetrasiloxane, and their mixtures.

**36.** Cosmetic process according to Claim 32 or 33, in which the liquid fatty phase comprises a non-volatile silicone oil.

**37.** Cosmetic process according to Claim 36, in which the non-volatile silicone oil is chosen from non-volatile polydialkylsiloxanes; polydimethylsiloxanes comprising pending alkyl, alkoxy or phenyl groups or alkyl, alkoxy or phenyl groups at the end of the silicone chain, which groups have from 2 to 24 carbon atoms; phenylated silicones; polysiloxanes modified with fatty acids (in particular $C_8$-$C_{20}$ fatty acids), fatty alcohols (in particular $C_8$-$C_{20}$) fatty alcohols) or polyoxyalkylenes (in particular polyoxyethylene and/or polyoxypropylene); aminated polysiloxanes; polysiloxanes comprising hydroxyl groups; fluorinated polysiloxanes comprising a pending fluorinated group or a fluorinated group at the end of the silicone chain having from 1 to 12 carbon atoms, all or part of the hydrogens of which are substituted by fluorine atoms; and their mixtures.

**38.** Cosmetic process according to any one of Claims 32 to 27, in which the liquid fatty phase comprises less than 50% by weight of non-silicone liquid organic compounds.

**39.** Cosmetic process according to any one of Claims 32 to 37, in which the liquid fatty phase does not comprise non-silicone liquid organic compounds.

**40.** Cosmetic process according to any one of Claims 32 to 39, in which the macromonomer is a silicone macromonomer.

**41.** Cosmetic process according to Claim 40, in which the silicone macromonomer is an organopolysiloxane macromonomer and preferably a polydimethylsiloxane macromonomer.

**42.** Cosmetic process according to any one of Claims 32 to 41, in which the grafted acrylic polymer is capable of being

obtained by radical polymerization in the said liquid fatty phase:

- of a main acrylic monomer chosen from $C_1$-$C_3$ alkyl (meth)acrylates, alone or as a mixture, and optionally of one or more additional acrylic monomers chosen from acrylic acid, methacrylic acid and alkyl (meth)acrylates of formula (VII):

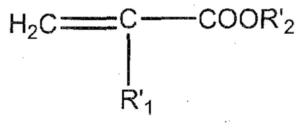

(VII)

in which:

- $R'_1$ denotes a hydrogen atom or a methyl group;
- $R'_2$ represents:
  - a linear or branched alkyl group comprising from 1 to 6 carbon atoms, the said group comprising, in its chain, one or more oxygen atoms and/or comprising one or more substituents chosen from -OH, halogen atoms (F, Cl, Br, I) and -NR'R" with R' and R", which are identical or different, chosen from linear or branched $C_1$-$C_3$ alkyls;
  - a cyclic alkyl group comprising from 3 to 6 carbon atoms, it being possible for the said group to comprise, in its chain, one or more oxygen atoms and/or it being possible for the said group to comprise one or more substituents chosen from OH and halogen atoms (F, Cl, Br, I);

and their salts, in order to form the said insoluble backbone;

- and of a silicone macromonomer.

**43.** Cosmetic process according to Claim 42, in which $R'_2$ denotes a group chosen from the methoxyethyl, ethoxyethyl, trifluoroethyl, 2-hydroxyethyl, 2-hydroxypropyl, dimethylaminoethyl, diethylaminoethyl and dimethylaminopropyl groups.

**44.** Cosmetic process according to Claim 42, in which the main acrylic monomer is chosen from methyl (meth)acrylate, ethyl (meth)acrylate, n-propyl meth(acrylate), isopropyl (meth)acrylate, and their mixtures.

**45.** Cosmetic process according to Claim 44, in which the main acrylic monomer is chosen from methyl acrylate, methyl methacrylate or ethyl methacrylate.

**46.** Cosmetic process according to Claim 42, in which the additional acrylic monomer is chosen from (meth)acrylic acid, methoxyethyl (meth)acrylate, ethoxyethyl (meth)acrylate, trifluoroethyl methacrylate, dimethylaminoethyl methacrylate, diethylaminoethyl methacrylate, 2-hydroxypropyl (meth)acrylate, 2-hydroxyethyl (meth)acrylate, their salts and their mixtures.

**47.** Cosmetic process according to Claim 46, in which the additional acrylic monomer is chosen from acrylic acid or methacrylic acid.

**48.** Cosmetic process according to any one of Claims 42 to 47, in which the silicone macromonomer is chosen from polydimethylsiloxanes comprising a mono(meth)acrylate end group.

**49.** Cosmetic process according to Claim 48,in which the silicone macromonomer corresponds to the following formula (VIII):

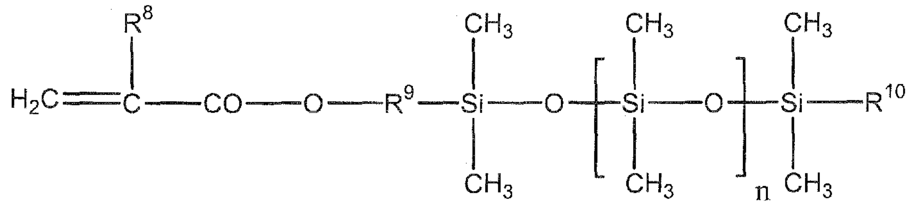

(VIII)

in which:

- $R^8$ represents a hydrogen atom or a methyl group;
- $R^9$ represents a divalent hydrocarbon group having from 1 to 10 carbon atoms and optionally comprising one or two ether -0- bonds;
- $R^{10}$ represents an alkyl group having from 1 to 10 carbon atoms, in particular from 2 to 8 carbon atoms;
- n represents an integer ranging from 1 to 300, preferably ranging from 3 to 200 and preferentially ranging from 5 to 100.

**50.** Cosmetic process according to any one of Claims 33 to 49, in which the dispersion is obtained by polymerization of methyl acrylate and of the macromonomer monomethacryloyloxypropylpolydimethylsiloxane in cyclopentadimethylsiloxane and the tensioning agent is a colloidal silica dispersion.

**51.** Cosmetic process according to any one of Claims 1 to 50, in which the grafted polymer has a weight-average molecular weight (Mw) of between 10 000 and 300 000, in particular between 20 000 and 200 000, better still between 25 000 and 150 000.

**52.** Cosmetic process according to any one of Claims 1 to 51, in which the grafted polymer particles have a mean size ranging from 10 to 400 nm, preferably ranging from 20 to 200 nm.

**53.** Cosmetic process according to any one one of Claims 1 to 52, in which the composition is applied to the outline of the eye.

**54.** Cosmetic process according to any one of Claims 1 to 53, in which the composition is a care composition or a make-up composition.

**55.** Cosmetic composition comprising, in a physiologically acceptable medium suitable for topical application to the skin of the face:

- from 0.1 to 20% by weight of at least one tensioning agent, with respect to the total weight of the composition, the said tensioning agent being in the form of colloidal particles of inorganic fillers; and
- at least one dispersion in a liquid fatty phase of solid particles of a grafted ethylenic polymer.

**56.** Use of a dispersion of solid particles of a grafted ethylenic polymer as defined according to any one of Claims 1 to 52 for improving the persistence of the tensioning effect provided by a tensioning agent as defined according to any one of Claims 1 to 7.

**57.** Use of a dispersion of solid particles of a grafted ethylenic polymer as defined according to any one of Claims 1 to 52 in a cosmetic composition comprising, as tensioning agent, an aqueous dispersion of colloidal inorganic particles, in particular of silica, for preventing whitening of the skin.

**Patentansprüche**

1. Kosmetisches Verfahren, um die Falten von Haut, die Falten aufweist, abzuschwächen, das einen Schritt aufweist, der darin besteht, auf die Haut, die Falten aufweist, eine kosmetische Zusammensetzung aufzutragen, die in einem physiologisch akzeptablen, für eine topische Anwendung auf die Gesichtshaut geeigneten Medium enthält:

   - 0,1 bis 20 Gew.-% mindestens eines straffenden Wirkstoffs, bezogen auf das Gesamtgewicht der Zusammensetzung, und
   - mindestens eine Dispersion von festen Partikeln eines gepfropften ethylenischen Polymers in einer flüssigen Fettphase.

2. Kosmetisches Verfahren nach Anspruch 1, wobei der straffende Wirkstoff in einem Mengenanteil von 1 bis 10 % des Gesamtgewichts der Zusammensetzung enthalten ist.

3. Kosmetisches Verfahren nach Anspruch 1 oder 2, wobei die Dispersion der Zusammensetzung in einem Mengenanteil von 0,01 bis 20 % und vorzugsweise 1 bis 10 % des Gesamtgewichts der Zusammensetzung vorliegt.

4. Kosmetisches Verfahren nach einem der Ansprüche 1 bis 3, wobei die flüssige Fettphase in der Zusammensetzung in einem Mengenanteil von 0,5 bis 80 % des Gesamtgewichts der Zusammensetzung, vorzugsweise 1 bis 55 % und noch bevorzugter 1 bis 25 % enthalten ist.

5. Kosmetisches Verfahren nach einem der Ansprüche 1 bis 4, wobei der straffende Wirkstoff ein Wirkstoff ist, der bei einer Konzentration von 7 % in Wasser bei einer Temperatur von 30 °C und unter einer relativen Feuchte von 40 % eine mit einem Extensometer gemessene Retraktion von isoliertem *Stratum corneum* von mehr als 1 % und vorzugsweise über 1,5 % hervorruft.

6. Kosmetisches Verfahren nach einem der Ansprüche 1 bis 5, wobei der straffende Wirkstoff unter den synthetischen Polymeren, Polymeren natürlicher Herkunft, gemischten Silicaten, Wachsmikropartikeln, kolloidalen Partikeln von anorganischen Füllstoffen und deren Gemischen ausgewählt ist.

7. Kosmetisches Verfahren nach Anspruch 6, wobei die synthetischen Polymere ausgewählt sind unter:

   - Polymeren und Copolymeren von Polyurethan;
   - Acrylpolymeren und Acrylcopolymeren;
   - Polymeren von sulfonierter Isophthalsäure;
   - gepfropften Siliconpolymeren;
   - wasserlöslichen oder in Wasser dispergierbaren Polymeren, die wasserlösliche Einheiten oder in Wasser dispergierbare Einheiten und LCST-Einheiten enthalten;
   - ethylenischen, sequentiellen, linearen, filmbildenden, nicht elastomeren und nicht wasserlöslichen Polymeren, die bei 1 Hz und 22 °C einen dynamischen Modul E' über 200 MPa aufweisen;
   - gepfropften ethylenischen Polymeren als Dispersion von festen Partikeln in einer flüssigen Fettphase, die eine Glasübergangstemperatur über 40 °C aufweisen
   - und deren Gemischen.

8. Kosmetisches Verfahren nach Anspruch 6, wobei die Polymere natürlicher Herkunft unter den pflanzlichen Proteinen und pflanzlichen Proteinhydrolysaten, Polysacchariden pflanzlicher Herkunft in Form von Mikrogelen, Latices pflanzlicher Herkunft und deren Gemischen ausgewählt sind.

9. Kosmetisches Verfahren nach einem der Ansprüche 1 bis 8, wobei das gepfropfte ethylenische Polymer ein in der flüssigen Fettphase unlösliches Grundgerüst und einen in der flüssigen Fettphase löslichen Teil aufweist, der aus Seitenketten besteht, die kovalent an das Grundgerüst gebunden sind.

10. Kosmetisches Verfahren nach einem der Ansprüche 1 bis 9, wobei das gepfropfte ethylenische Polymer ein gepfropftes Acrylpolymer ist.

11. Kosmetisches Verfahren nach Anspruch 10, wobei das gepfropfte Acrylpolymer durch radikalische Polymerisation der folgenden Monomere in der flüssigen Fettphase erhältlich ist:

- mindestens eines Acrylmonomers und gegebenenfalls mindestens eines ergänzenden, nicht acrylischen Vinylmonomers zur Bildung des unlöslichen Grundgerüsts; und
- mindestens eines Makromers, das eine polymerisierbare Endgruppe aufweist, zur Bildung der Seitenketten, wobei das Makromer eine gewichtsmittlere Molmasse von 200 oder darüber aufweist und der Gehalt des polymerisierbaren Makromers 0,05 bis 20 Gew.-% des Polymers ausmacht.

**12.** Kosmetisches Verfahren nach Anspruch 11, wobei das Acrylmonomer oder die Acrylmonomere unter den Monomeren ausgewählt sind, deren Homopolymer in der jeweiligen flüssigen Fettphase unlöslich ist, d. h., das Homopolymer liegt bei einer Konzentration von 5 Gew.-% oder darüber bei Raumtemperatur (20 °C) in der flüssigen Fettphase in fester Form vor.

**13.** Kosmetisches Verfahren nach Anspruch 11 oder 12, wobei das Acrylmonomer oder die Acrylmonomere unter den folgenden Monomeren ausgewählt sind:

- (Meth)acrylaten der folgenden Formel (IV):

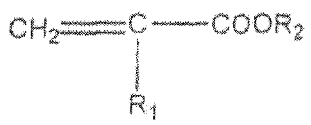

(IV)

worin bedeuten:

- $R_1$ ein Wasserstoffatom oder eine Methylgruppe;
- $R_2$ eine Gruppe, die ausgewählt ist unter:
- einer geradkettigen oder verzweigten Alkylgruppe, die 1 bis 6 Kohlenstoffatome aufweist, wobei die Gruppe in ihrer Kette ein oder mehrere Heteroatome enthalten kann, die unter O, N und S ausgewählt sind; und/oder einen oder mehrere Substituenten aufweisen kann, die unter OH, Halogenatomen (F, Cl, Br, I) und -NR'R" ausgewählt sind, wobei R' und R", die gleich oder verschieden sind, unter den geradkettigen oder verzweigten Alkylgruppen mit 1 bis 4 Kohlenstoffatomen ausgewählt sind; und/oder mit mindestens einer Polyoxyalkylengruppe und insbesondere einer Alkylengruppe mit 1 bis 4 Kohlenstoffatomen, besonders einer Polyoxyethylengruppe und/oder Polyoxypropylengruppe substituiert sein kann, wobei die Polyoxyalkylengruppe durch Wiederholung von 5 bis 30 Oxyalkyleneinheiten gebildet ist;
- einer cyclischen Alkylgruppe, die 3 bis 6 Kohlenstoffatome aufweist, wobei die Gruppe in ihrer Kette ein oder mehrere Heteroatome enthalten kann, die unter O, N und S ausgewählt sind, und einen oder mehrere Substituenten aufweisen kann, die unter OH und Halogenatomen (F, Cl, Br, I) ausgewählt sind;
- (Meth)acrylamiden der folgenden Formel (V):

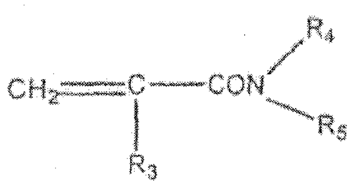

(V)

worin bedeuten:

- $R_3$ ein Wasserstoffatom oder eine Methylgruppe;
- $R_4$ und $R_5$, die gleich oder verschieden sind, ein Wasserstoffatom oder eine geradkettige oder verzweigte Alkylgruppe mit 1 bis 6 Kohlenstoffatomen, die einen oder mehrere Substituenten aufweisen kann, die unter -OH, Halogenatomen (F, Cl, Br, I) und -NR'R" ausgewählt sind, wobei R' und R", die gleich oder verschieden sind, unter geradkettigen oder verzweigten Alkylgruppen mit 1 bis 4 Kohlenstoffatomen ausgewählt sind;
- $R_4$ ein Wasserstoffatom und $R_5$ eine 1,1-Dimethyl-3-oxobutylgruppe;
- (Meth)acrylmonomeren, die mindestens eine Carbonsäurefunktion, Phosphorsäurefunktion oder Sulfonsäurefunktion besitzen;

wobei diese Monomere in Form ihrer Salze vorliegen können.

**14.** Kosmetisches Verfahren nach einem der Ansprüche 1 bis 13, wobei das oder die Vinylmonomere, die nicht auf Acrylmonomeren basieren, ausgewählt sind unter:

- Vinylestern der folgenden Formel (VI):

$$R_6\text{-COO-CH=CH}_2 \qquad (VI)$$

worin bedeuten:

- $R_6$ eine geradkettige oder verzweigte Alkylgruppe mit 1 bis 6 Kohlenstoffatomen oder eine cyclische Alkylgruppe mit 3 bis 6 Kohlenstoffatomen und/oder eine aromatische Gruppe, beispielsweise vom Benzoltyp, Anthracentyp und Naphthalintyp;
- Vinylmonomeren, die nicht auf Acrylmonomeren basieren und mindestens eine Carbonsäurefunktion oder Sulfonsäurefunktion aufweisen, wie Crotonsäure, Maleinsäureanhydrid, Itaconsäure, Fumarsäure, Maleinsäure, Styrolsulfonsäure, Vinylbenzoesäure, Vinylphosphorsäure und deren Salzen;
- Vinylmonomeren, die nicht auf Acrylmonomeren basieren und mindestens eine tertiäre Aminofunktion besitzen, wie 2-Vinylpyridin, 4-Vinylpyridin;
- und deren Gemischen.

**15.** Kosmetisches Verfahren nach einem der Ansprüche 11 bis 14, wobei das oder die Acrylmonomere 50 bis 100 Gew.-%, vorzugsweise 55 bis 100 Gew.-% und noch bevorzugter 60 bis 100 Gew.-% des Gemisches ausmachen, das aus dem oder den Acrylmonomeren und dem oder den gegebenenfalls enthaltenen nicht-acrylischen Vinylmonomeren besteht.

**16.** Kosmetisches Verfahren nach einem der Ansprüche 11 bis 13, wobei das gepfropfte Acrylpolymer keine zusätzlichen nicht-acrylischen Vinylmonomere enthält.

**17.** Kosmetisches Verfahren nach einem der Ansprüche 11 bis 16, wobei das Makromer an einem seiner Enden eine polymerisierbare Endgruppe aufweist, die unter einer Vinylgruppe, einer (Meth)acrylatgruppe ausgewählt ist.

**18.** Kosmetisches Verfahren nach einem der Ansprüche 11 bis 17, wobei das Makromer eine gewichtsmittlere Molmasse (Mw) von 200 bis 100 000, vorzugsweise 500 bis 50 000, noch bevorzugter 800 bis 20 000, besonders bevorzugt 800 bis 10 000 und ganz besonders bevorzugt 800 bis 6 000 aufweist.

**19.** Kosmetisches Verfahren nach einem der Ansprüche 11 bis 18, wobei das polymerisierte Makromer 0,1 bis 15 Gew.-% des Gesamtgewichts des Polymers, vorzugsweise 0,2 bis 10 Gew.-% und noch bevorzugter 0,3 bis 8 Gew.-% ausmacht.

**20.** Kosmetisches Verfahren nach einem der Ansprüche 11 bis 19, wobei das Makromer unter den Makromeren ausgewählt ist, deren Homopolymer in der jeweiligen flüssigen Fettphase löslich ist, d. h. bei einer Konzentration von 5 Gew.-% oder darüber und bei Raumtemperatur (20 °C) in der flüssigen Fettphase vollständig gelöst vorliegt.

**21.** Kosmetisches Verfahren nach einem der Ansprüche 1 bis 20, wobei die flüssige Fettphase mindestens eine nichtwässrige flüssige Verbindung enthält, die ausgewählt ist unter:

- organischen flüssigen Verbindungen mit einem Gesamtlöslichkeitsparameter nach dem Solubilitätsraum nach Hansen von 18 $(MPa)^{1/2}$ oder darunter und vorzugsweise höchstens 17 $(MPa)^{1/2}$;
- Monoalkoholen mit einem Gesamtlöslichkeitsparameter nach dem Solubilitätsraum nach Hansen von 20

(MPa)$^{1/2}$ oder darunter; und
- deren Gemischen.

**22.** Kosmetisches Verfahren nach Anspruch 21, wobei die flüssige Fettphase eine nichtsiliconierte Fettphase ist, wobei die nichtsiliconierte Fettphase keine flüssigen organischen Siliconverbindungen enthält oder weniger als 50 Gew.-% organische flüssige Siliconverbindungen enthält.

**23.** Kosmetisches Verfahren nach Anspruch 22, wobei die nichtsiliconierte Fettphase mindestens 50 Gew.-% mindestens einer organischen, nichtsiliconierten flüssigen Verbindung enthält, die ausgewählt ist unter:

- nichtsiliconierten organischen flüssigen Verbindungen mit einem Gesamtlöslichkeitsparameter nach dem Solubilitätsraum von Hansen von 18 (MPa)$^{1/2}$ oder darunter, vorzugsweise höchstens 17 (MPa)$^{1/2}$;
- flüssigen Monoalkoholen mit einem Gesamtlöslichkeitsparameter nach dem Solubilitätsraum nach Hansen von 20 (MPa)$^{1/2}$ oder darunter; und
- deren Gemischen.

**24.** Kosmetisches Verfahren nach Anspruch 23, wobei die nichtsiliconierte flüssige Verbindung mit einem Gesamtlöslichkeitsparameter nach dem Solubilitätsraum nach Hansen von höchstens 18 (MPa) $^{1/2}$ und vorzugsweise höchstens 17 (MPa)$^{1/2}$ ausgewählt ist unter:

- Ölen auf Kohlenstoffbasis, Ölen auf Kohlenwasserstoffbasis, fluorierten Ölen, die gegebenenfalls verzweigt sind, die natürlich oder synthetisch sind, einzeln oder als Gemisch;
- geradkettigen, verzweigten und/oder cyclischen, gegebenenfalls flüchtigen Alkanen;
- Estern und insbesondere linearen, verzweigten oder cyclischen Estern mit mindestens 6 Kohlenstoffatomen und insbesondere 6 bis 30 Kohlenstoffatomen;
- Ethern und insbesondere Ethern mit mindestens 6 Kohlenstoffatomen und insbesondere 6 bis 30 Kohlenstoffatomen;
- Ketonen und insbesondere Ketonen mit mindestens 6 Kohlenstoffatomen und besonders 6 bis 30 Kohlenstoffatomen.

**25.** Kosmetisches Verfahren nach Anspruch 23, wobei die Monoalkohole mit einem Gesamtlöslichkeitsparameter nach dem Solubilitätsraum nach Hansen von höchstens 20 (MPa)$^{1/2}$ ausgewählt sind unter den aliphatischen, gesättigten oder ungesättigten flüssigen Monoalkoholen mit mindestens 6 Kohlenstoffatomen.

**26.** Kosmetisches Verfahren nach einem der Ansprüche 23 bis 25, wobei das oder die Makromere Makromere auf Kohlenstoffbasis sind.

**27.** Kosmetisches Verfahren nach Anspruch 26, wobei das Makromer auf Kohlenstoffbasis ausgewählt ist unter:

- (i) Homopolymeren und Copolymeren von Alkyl(meth)acrylat mit linearer oder verzweigter $C_{8-22}$-Alkylgruppe, die eine polymerisierbare endständige Gruppe aufweisen, die unter Vinyl oder (Meth)acrylat ausgewählt ist;
- (ii) Polyolefinen mit einer endständigen Gruppe mit ethylenisch ungesättigter Bindung, insbesondere mit einer endständigen (Meth)acrylatgruppe.

**28.** Kosmetisches Verfahren nach Anspruch 26, wobei das Makromer auf Kohlenstoffbasis ausgewählt ist unter:

- (i) Makromeren von Poly-(2-ethylhexylacrylat) mit endständigem Mono(meth)acrylat; Makromeren von Poly (dodecylacrylat) mit endständigem Mono(meth)acrylat; Makromeren von Poly(dodecylmethacrylat); Makromeren von Poly(stearylacrylat) mit endständigem Mono(meth)acrylat; Makromeren von Poly(stearylmethacrylat) mit endständigem Mono(meth)acrylat;
- (ii) Makromeren von Polyethylen, Makromeren von Polypropylen, Makromeren eines Polyethylen/Polypropylen-Copolymers, Makromeren eines Polyethylen/Polybutylen-Copolymers, Makromeren von Polyisobutylen, Makromeren von Polybutadien, Makromeren von Polyisopren, Makromeren von Polybutadien, Makromeren von Poly(ethylen/butylen)-polyisopren, wobei diese Makromere eine endständige (Meth)acrylatgruppe aufweisen.

**29.** Kosmetisches Verfahren nach Anspruch 27 oder 28, wobei das Makromer auf Kohlenstoffbasis ausgewählt ist unter:

- (i) Makromeren von Poly(2-ethylhexylacrylat) mit endständigem Mono(meth)acrylat, Makromeren von Poly (dodecylacrylat) mit endständigem Mono(meth)acrylat;
- (ii) Poly(ethylen/butylen)-methacrylat.

**30.** Kosmetisches Verfahren nach einem der Ansprüche 1 bis 29, wobei die Dispersion eine Dispersion ist, die durch Polymerisation von Methylacrylat und einem Polyethylen/Polybutylen-Methacrylat in Isododecan erhalten wird, und das Straffungsmittel eine kolloidale Dispersion von Kieselsäure ist.

**31.** Kosmetisches Verfahren nach einem der Ansprüche 1 bis 29, wobei die Dispersion eine Dispersion ist, die durch Polymerisation von Methylacrylat und einem Polyethylen/Polybutylen-Methacrylatmakromer in Isododecan gebildet wird, und das Straffungsmittel eine Dispersion ist, die durch Polymerisation von Methylacrylat, Acrylsäure und einem Polyethylen/Polybutylen-Methacrylatmakromer in Isododecan gebildet wird.

**32.** Kosmetisches Verfahren nach Anspruch 21, wobei die flüssige Fettphase eine flüssige siliconierte Fettphase ist.

**33.** Kosmetisches Verfahren nach Anspruch 32, wobei die siliconierte flüssige Fettphase mindestens 50 Gew.-% mindestens einer flüssigen organischen Siliconverbindung enthält, die unter den flüssigen organischen Siliconverbindungen mit einem Gesamtlöslichkeitsparameter nach dem Solubilitätsraum von Hansen von 17 $(MPa)^{1/2}$ oder darunter ausgewählt ist.

**34.** Kosmetisches Verfahren nach Anspruch 32 oder 33, wobei die flüssige Fettphase ein flüchtiges Siliconöl enthält.

**35.** Kosmetisches Verfahren nach Anspruch 34, wobei das flüchtige Siliconöl unter Octamethylcyclotetrasiloxan, Decamethylcyclopentasiloxan, Dodecymethylcyclohexasiloxan, Heptamethylhexyltrisiloxan, Heptamethyloctyltrisiloxan, Octamethyltrisiloxan, Decamethyltetrasiloxan und deren Gemischen ausgewählt ist.

**36.** Kosmetisches Verfahren nach Anspruch 32 oder 33, wobei die flüssige Fettphase ein nichtflüchtiges Siliconöl enthält.

**37.** Kosmetisches Verfahren nach Anspruch 36, wobei das nichtflüchtige Siliconöl ausgewählt ist unter: nichtflüchtigen Polydialkylsiloxanen; Polydimethylsiloxanen, die als Seitengruppen oder Siliconkette Alkyl-, Alkoxy- oder Phenylgruppen enthalten, wobei diese Gruppen 2 bis 24 Kohlenstoffatome aufweisen; phenylierten Siliconen; Polysiloxanen, die mit $C_8$-$_{20}$-Fettsäuren, $C_{8-20}$-Fettalkoholen oder Polyoxyalkylenen (insbesondere Polyoxyethylen und/oder Polyoxypropylen) modifiziert sind; aminierten Polysiloxanen; Polysiloxanen mit Hydroxygruppen; fluorierten Polysiloxanen, die als Seitenkette oder am Ende der Siliconkette eine fluorierte Gruppe mit 1 bis 12 Kohlenstoffatomen aufweist, bei der alle Wasserstoffatome oder ein Teil der Wasserstoffatome durch Fluoratome ersetzt sind; und deren Gemischen.

**38.** Kosmetisches Verfahren nach einem der Ansprüche 32 bis 37, wobei die flüssige Fettphase weniger als 50 Gew.-% nichtsiliconierte flüssige organische Verbindungen enthält.

**39.** Kosmetisches Verfahren nach einem der Ansprüche 32 bis 37, wobei die flüssige Fettphase keine nichtsiliconierten organischen Verbindungen enthält.

**40.** Kosmetisches Verfahren nach einem der Ansprüche 32 bis 39, wobei das Makromer ein Siliconmakromer ist.

**41.** Kosmetisches Verfahren nach Anspruch 40, wobei das Siliconmakromer ein Organopolysiloxanmakromer und vorzugsweise ein Polydimethylsiloxanmakromer ist.

**42.** Kosmetisches Verfahren nach einem der Ansprüche 32 bis 41, wobei das gepfropfte Acrylpolymer durch radikalische Polymerisation der folgenden Monomere in einer flüssigen Fettphase erhältlich ist:

eines hauptsächlich vorliegenden Acrylmonomers, das unter den Alkyl($C_{1-3}$)(meth)acrylaten oder deren Gemischen ausgewählt ist, und gegebenenfalls eines oder mehrerer ergänzender Acrylmonomere, die unter Acrylsäure, (Meth)acrylsäure und Alkyl(meth)acrylaten der Formel (VII) ausgewählt sind:

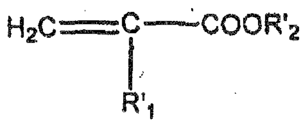

(VII)

worin bedeuten:

- $R'_1$ ein Wasserstoffatom oder eine Methylgruppe;
- $R'_2$:
- eine geradkettige oder verzweigte Alkylgruppe mit 1 bis 6 Kohlenstoffatomen, wobei diese Gruppe in ihrer Kette ein oder mehrere Sauerstoffatome aufweist und/oder einen oder mehrere Substituenten enthält, die ausgewählt sind unter -OH, Halogenatomen (F, Cl, Br, I) und -NR'R", wobei die Gruppen R' und R", die gleich oder verschieden sind unter den geradkettigen oder verzweigten $C_{1-3}$-Alkylgruppen ausgewählt sind;
- eine cyclische Alkylgruppe, die 3 bis 6 Kohlenstoffatome aufweist, wobei diese Gruppe in ihrer Kette ein oder mehrere Sauerstoffatome enthalten kann und/oder einen oder mehrere Substituenten aufweisen kann, die unter OH und den Halogenatomen (F, Cl, Br, I) ausgewählt sind;

und deren Salzen, um das unlösliche Grundgerüst zu bilden;

- und ein Siliconmakromonomer.

**43.** Kosmetisches Verfahren nach Anspruch 42, wobei $R'_2$ eine Gruppe bedeutet, die unter Methoxyethyl, Ethoxymethyl, Trifluorethyl; 2-hydroxymethyl, 2-Hydroxypropyl, Dimethylaminoethyl, Diethylaminoethyl, Dimethylaminopropyl ausgewählt ist.

**44.** Kosmetisches Verfahren nach Anspruch 42, wobei das hauptsächlich vorliegende Acrylmonomer unter Methyl(meth)acrylat, Ethyl(meth)acrylat, n-Propyl(meth)acrylat, Isopropyl(meth)acrylat und deren Gemischen ausgewählt ist.

**45.** Kosmetisches Verfahren nach Anspruch 44, wobei das hauptsächlich vorliegende Acrylmonomer unter Methylacrylat, Methylmethacrylat und Ethylmethacrylat ausgewählt ist.

**46.** Kosmetisches Verfahren nach Anspruch 42, wobei das ergänzende Acrylmonomer ausgewählt ist unter (Meth)acrylsäure, Methoxyethyl(meth)acrylat, Ethoxyethyl(meth)acrylat, Trifluorethyl(meth)acrylat, Dimethylaminoethylmethacrylat, Diethylaminoethylmethacrylat, 2-Hydroxypropyl(meth)acrylat, 2-Hydroxyethyl(meth)acrylat und deren Salzen und deren Gemischen.

**47.** Kosmetisches Verfahren nach Anspruch 46, wobei das ergänzende Acrylmonomer unter Acrylsäure und Methacrylsäure ausgewählt ist.

**48.** Kosmetisches Verfahren nach einem der Ansprüche 42 bis 47, wobei das Siliconmakromonomer unter Polydimethylsiloxanen mit endständiger Mono(meth)acrylatgruppe ausgewählt ist.

**49.** Kosmetisches Verfahren nach Anspruch 48, wobei das Siliconmakromonomer der folgenden Formel (VIII) entspricht:

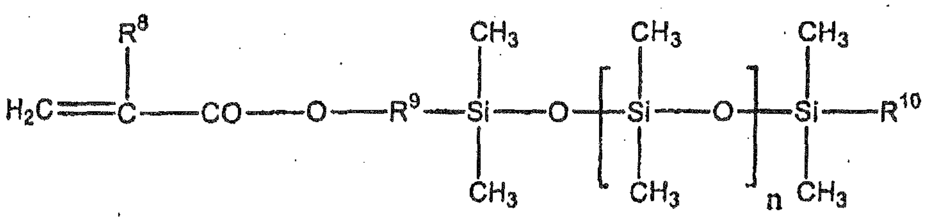

(VIII)

worin bedeuten:

- $R^8$ ein Wasserstoffatom oder eine Methylgruppe;
- $R^9$ eine zweiwertige Kohlenwasserstoffgruppe mit 1 bis 10 Kohlenstoffatomen, die gegebenenfalls eine oder mehrere Etherbindungen -O- aufweist;
- $R^{10}$ eine Alkylgruppe mit 1 bis 10 Kohlenstoffatomen und insbesondere 2 bis 8 Kohlenstoffatomen;
- n eine ganze Zahl von 1 bis 300, vorzugsweise 3 bis 200 und besonders bevorzugt 5 bis 100.

**50.** Kosmetisches Verfahren nach einem der Ansprüche 33 bis 49, wobei die Dispersion durch Polymerisation von Methylacrylat und einem Monomethacryloxypropylpolydimethylsiloxanmakromonomer in Cyclopentadimethylsiloxan erhalten wird und das Straffungsmittel eine kolloidale Dispersion von Kieselsäure ist.

**51.** Kosmetisches Verfahren nach einem der Ansprüche 1 bis 50, wobei das gepfropfte Polymere eine gewichtsmittlere Molmasse (Mw) von 10 000 bis 300 000, insbesondere 20 000 bis 200 000 und noch besser im Bereich von 25 000 bis 150 000 aufweist.

**52.** Kosmetisches Verfahren nach einem der Ansprüche 1 bis 51, wobei die Partikel des gepfropften Polymers eine mittlere Größe von 10 bis 400 nm und vorzugsweise 20 bis 200 nm aufweisen.

**53.** Kosmetisches Verfahren nach einem der Ansprüche 1 bis 52, wobei die Zusammensetzung auf die Augenkonturen aufgebracht wird.

**54.** Kosmetisches Verfahren nach einem der Ansprüche 1 bis 53, wobei die Zusammensetzung eine Zusammensetzung für die Pflege oder eine Zusammensetzung zum Schminken ist.

**55.** Kosmetische Zusammensetzung, die in einem physiologisch akzeptablen, für eine topische Anwendung auf das Gesicht geeigneten Medium enthält:

- 0,1 bis 20 Gew.-% mindestens eines Straffungsmittels, bezogen auf das Gesamtgewicht der Zusammensetzung, wobei das Straffungsmittel in Form von kolloidalen Partikeln von anorganischen Füllstoffen vorliegt; und
- mindestens eine Dispersion von festen Partikeln eines ethylenischen gepfropften Polymers in einer flüssigen Fettphase.

**56.** Verwendung einer Dispersion von festen Partikeln eines gepfropften ethylenischen Polymers, wie es in einem der Ansprüche 1 bis 52 definiert ist, um die Remanenz der straffenden Wirkung zu verbessern, die durch einen straffenden Wirkstoff hervorgerufen wird, wie er in einem der Ansprüche 1 bis 7 definiert ist.

**57.** Verwendung einer Dispersion von festen Partikeln eines gepfropften ethylenischen Polymers nach einem der Ansprüche 1 bis 52 in einer kosmetischen Zusammensetzung, die als straffenden Wirkstoff eine wässrige Dispersion von anorganischen kolloidalen Partikeln, insbesondere von Kieselsäure, enthält, um dem Weißwerden der Haut vorzubeugen.

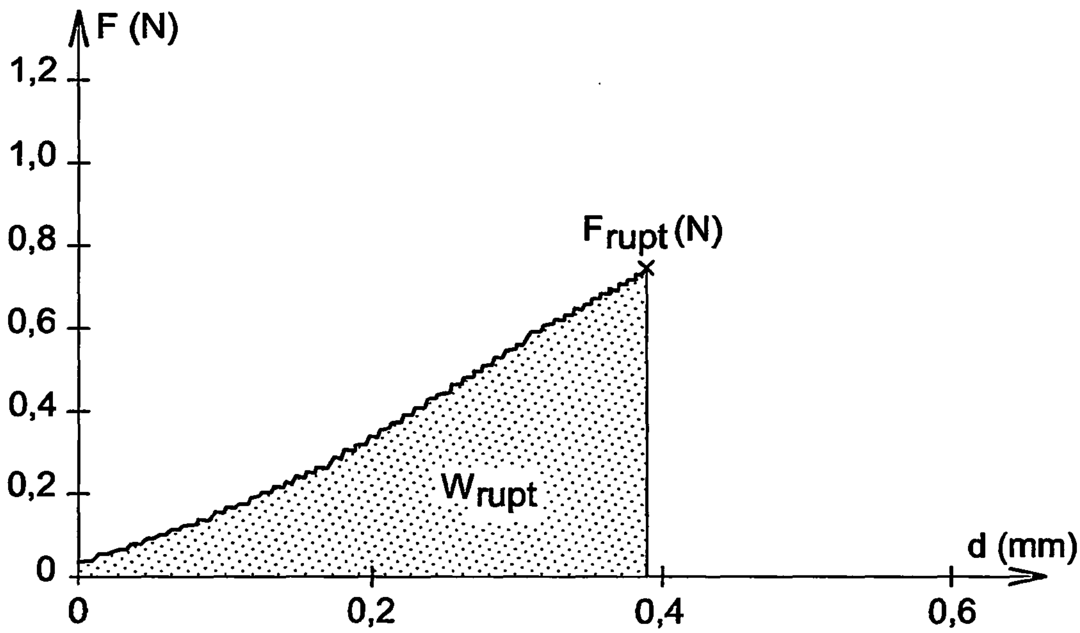
F (N)
1,2
1,0
0,8
0,6
0,4
0,2
0
Frupt (N)
Wrupt
d (mm)
0
0,2
0,4
0,6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2758083 A
- EP 1038519 A
- US 6139322 A
- US 6465001 B
- US 5349003 A
- FR 2819429
- FR 0311346
- FR 2829025
- EP 895467 A
- EP 96459 A
- US 5625005 A


### Littérature non-brevet citée dans la description


- KIRK-OTHMER ENCYCLOPEDIA OF CHEMICAL TECHNOLOGY. Wiley Interscience, 1983, vol. 21, 492-507
- **VAN DE HULST, H.C.** Light Scattering by Small Particles. Wiley, 1957
- Solubility parameter values. **ERIC A.GRULKE.** Polymer Handbook. 519-559
- **C.M.HANSEN.** The three dimensional solubility parameters. *J.Paint Technol.,* 1967, vol. 39, 105
- **GILLMAN K.F.** *Polymer Letters,* 1967, vol. 5, 477-481
- **PETER HARRIS.** Food Gels. Elsevier, 1989
- Food Gels. Elsevier, 1989
- Kirk-Othmer Encyclopedia Of Chemical Technology. Wiley Interscience, 1983, vol. 21, 492-507
- Light Scattering by Small Particles. Wiley, 1957
- Polymer Handbook. 519-559
- *J.Paint Technol.,* 1967, vol. 39, 105
- *Polymer Letters,* 1967, vol. 5, 477-481